# EUROPEAN PATENT APPLICATION

(11) **EP 4 129 275 A1**
(43) Date of publication of application: **08.02.2023**
(21) Application number: 21779530.1
(22) Date of filing: 30.03.2021
(51) Int. Cl.: A61K 31/045, A23L 5/00, A24B 15/00, A24D 3/00, A61K 9/08, A61K 9/48, A61K 9/72, A61K 47/08, A61K 47/10, A61K 47/14, A61K 47/18, A61K 47/46, A61P 1/00, A61Q 13/00

(54) **COMPOSITION CONTAINING MENTHOL**

(30) Priority: 30.03.2020 US 202063001679 P; 04.08.2020 US 202063060776 P
(71) Applicant: Sunsho Pharmaceutical Co., Ltd., Fuji-shi, Shizuoka 419-0201 (JP)
(72) Inventor: YAMADA, Kazuya, Fuji-shi, Shizuoka 419-0201 (JP); SUZUKI, Tomoya, Fuji-shi, Shizuoka 419-0201 (JP); ITO, Masakazu, Fuji-shi, Shizuoka 419-0201 (JP); DOBASHI, Kouichi, Fuji-shi, Shizuoka 419-0201 (JP)
(74) Representative: Lederer & Keller Patentanwälte Partnerschaft mbB
(86) International application number: PCT/JP2021/013731
(87) International publication number: WO 2021/201059

(57) **Abstract**

An object of the present invention is to provide is a novel composition and others. The composition comprises l-menthol and at least one ingredient (X) selected from dicarboxylic acid esters, diol esters, monocarboxylic acid esters, esters of polyols having three or more hydroxy groups, esters of polycarboxylic acids having three or more carboxyl groups, polyol ethers, polyamines, and alcohols having six or more carbon atoms.

## Description

### TECHNICAL FIELD

The present invention relates to an l-menthol-containing composition and others (e.g., a technique for dissolving a high concentration of l-menthol in a composition).

### BACKGROUND ART

l-Menthol is a compound typically providing a cooling sensation and has been used in chewing gums, mouth fresheners, and tobacco flavoring agents. In order to enhance the cooling sensation, a high-concentration l-menthol solution is required, but l-menthol is lipophilic and highly crystalline, thus being difficult to dissolve at a high concentration in a solution.

Meanwhile, attempts have been made to dissolve l-menthol using various additives. For example, a report shows that an emulsifier having a hydrophilic-lipophilic balance (HLB) value of 10 to 16 and propylene glycol at a concentration of 30 wt% or more are used to prevent menthol recrystallization after formation of tablets containing 50 wt% or less menthol (Patent Literature 1). In other reports, 2 to 50 parts by weight of d-neomenthol relative to 100 parts by weight of l-menthol is used to prevent crystal precipitation of l-menthol (Patent Literature 2 and 3) . In another report, 0.1 to 0.5 mass% sugar fatty acid ester, a terpene compound that is liquid at 25_{°}C, a carboxylic acid ester compound having a total of 12 to 18 carbon atoms, and a polyglycerol fatty acid ester which is a monoester of a polyglycerol having 5 to 10 repeating units and a fatty acid having 12 to 14 carbon atoms are used in combination to prevent crystal precipitation of l-menthol (Patent Literature 4). In yet another report, lecithin and/or an organic acid monoglyceride, triglyceride, and ghatti gum are used in combination to prevent crystal precipitation of l-menthol (Patent Literature 5).

On the other hand, among solvents that are highly able to dissolve l-menthol, medium chain triglycerides (MCTs) reportedly enable better dissolution of l-menthol than corn oil, peanut oil, sunflower oil, canola oil, and glycerol. According to this report, an MCT solution of l-menthol at a concentration of up to 48.7 wt% is a stable liquid at 20_{°}C (Patent Literature 6).

### CITATION LIST

### Patent Literature

Patent Literature 1: Japanese Patent No. 3094207
Patent Literature 2: JP-A 2010-051246
Patent Literature 3: Japanese Patent No. 4750162
Patent Literature 4: Japanese Patent No. 4861650
Patent Literature 5: Japanese Patent No. 5871609
Patent Literature 6: WO 2014/117265

### SUMMARY OF INVENTION

### TECHNICAL PROBLEM

However, emulsifiers such as sugar fatty acid esters and fatty acid monoesters are avoided in some applications. For example, when capsules containing a high concentration of menthol are produced, such an emulsifier in the formula can disturb encapsulation. In addition, d-neomenthol is not commonly available and can only be obtained by extraction or synthesis, and thus using d-neomenthol would be very costly. Furthermore, MCTs have limitations in enhancing dissolution of l-menthol by themselves and are expensive as well; and thus alternative ingredients to MCTs or ingredients that can be partially replaced with MCTs are required.

In response to this issue, an object of the present invention is to provide an ingredient (novel ingredient) useful for dissolving (preventing crystallization of) l-menthol, and others.

### SOLUTION TO PROBLEM

After extensive research to achieve the above-mentioned object, the present inventors found that specific ingredients or ingredients identified based on specific indices (such as a water/octanol partition coefficient) are useful for dissolving l-menthol (hereinafter sometimes referred to simply as menthol) (can facilitate, improve or enhance dissolution of menthol) . Based on these findings, the present inventors have completed the present invention.

That is, the present invention relates to the following.
[1] A composition comprising l-menthol and at least one ingredient (X) selected from dicarboxylic acid esters, diol esters, monocarboxylic acid esters, esters of polyols having three or more hydroxy groups, esters of polycarboxylic acids having three or more carboxyl groups, polyol ethers, polyamines, and alcohols having six or more carbon atoms.
[2] A composition comprising l-menthol and an ingredient (X) satisfying at least one selected from the following requirements (1) and (2):
   (1) the ingredient (X) has a water/octanol partition coefficient (log P) of 10 or less; and
   (2) the ingredient (X) has a hydrophilic-lipophilic balance (HLB) value of 12 or less.
[3] A composition comprising l-menthol and at least one ingredient (X) selected from dicarboxylic acid esters, diol esters, monocarboxylic acid esters, esters of polyols having three or more hydroxy groups, esters of polycarboxylic acids having three or more carboxyl groups, polyol ethers, polyamines, and alcohols having six or more carbon atoms, wherein the ingredient (X) satisfies at least one selected from the following requirements (1) and (2):
   (1) the ingredient (X) has a water/octanol partition coefficient (log P) of 10 or less; and
   (2) the ingredient (X) has a hydrophilic-lipophilic balance (HLB) value of 12 or less.
[4] The composition according to the above [2] or [3], wherein the ingredient (X) satisfies at least the requirement (1).
[5] The composition according to any one of the above [2] to [4], wherein the ingredient (X) satisfies the requirements (1) and (2).
[6] The composition according to any one of the above [2] to [5], wherein the ingredient (X) has a log P of 0.3 to 8 and/or an HLB value of 9 or less.
[7] The composition according to any one of the above [1] to [6], wherein the ingredient (X) comprises at least one selected from dicarboxylic acid diesters, diol diesters, monocarboxylic acid esters, triol triesters, tricarboxylic acid triesters, diol diethers, and diamines.
[8] The composition according to any one of the above [1] to [7], wherein the ingredient (X) comprises at least one selected from aliphatic dicarboxylic acid diesters, diesters of aliphatic diols having three or more carbon atoms, aliphatic monocarboxylic acid esters, aliphatic triol tri-C₁₋₆ fatty acid esters, aliphatic diol diethers, and N-alkyl substituted diamines.
[9] The composition according to any one of the above [1] to [8], wherein the ingredient (X) comprises an aliphatic dicarboxylic acid diester.
[10] The composition according to any one of the above [1] to [9], wherein the ingredient (X) comprises a diester of an aliphatic dicarboxylic acid having four or more carbon atoms.
[11] The composition according to any one of the above [1] to [10], wherein the ingredient (X) comprises at least one selected from succinic acid diesters (e.g., dialkyl succinates such as diethyl succinate), adipic acid diesters (e.g., dialkyl adipates such as diisobutyl adipate), and sebacic acid diesters (e.g., dialkyl adipates such as diethyl sebacate).
[12] The composition according to any one of the above [1] to [11], wherein the ingredient (X) comprises at least one selected from 1-decanol, 1-dodecanol, 1-hexadecanol, and benzyl alcohol.
[13] The composition according to any one of the above [1] to [12], wherein the ingredient (X) comprises at least one selected from 1-dodecanol and 1-hexadecanol.
[14] The composition according to any one of the above [1] to [13], wherein the composition comprises
   an alcohol having six or more carbon atoms, and
   a fat or oil (e.g., a medium chain triglyceride (MCT)) and/or an ingredient (X) other than the alcohol having six or more carbon atoms.
[15] The composition according to any one of the above [1] to [14], wherein the composition comprises
   at least one selected from C₁₀₋₁₆ alkanols and aralkyl alcohols, and
   at least one selected from dicarboxylic acid esters (particularly, aliphatic dicarboxylic acid diesters) and fats or oils (particularly, MCTs).
[16] The composition according to any one of the above [1] to [15], wherein the concentration of the l-menthol is 20 mass% or more (e.g., 25 mass% or more).
[17] The composition according to any one of the above [1] to [16], wherein the concentration of the l-menthol is 30 mass% or more [e.g., 35 mass% or more (e.g., 35 to 80 mass%)].
[18] The composition according to any one of the above [1] to [17], wherein the concentration of the l-menthol is 50 mass% or more (e.g., 55 mass% or more).
[19] The composition according to any one of the above [1] to [18], wherein the amount of the ingredient (X) is 80 mass% or less (e.g., 75 mass% or less) of the combined amount of the l-menthol and the ingredient (X).
[20] The composition according to any one of the above [1] to [19], wherein the amount of the ingredient (X) is 50 mass% or less (e.g., 45 mass% or less) of the combined amount of the l-menthol and the ingredient (X).
[21] The composition according to any one of the above [1] to [20], further comprising a fat or oil (particularly, an MCT) .
[22] The composition according to any one of the above [1] to [21], wherein the composition is a pharmaceutical composition.
[23] A capsule having a core (content, inner liquid) and a shell, wherein the core (content) is formed of the composition according to any one of the above [1] to [22] (or wherein the core contains l-menthol and an ingredient (X)).
[24] A filter at least containing a (first) capsule (a capsule-embedded filter, a filter composed of a capsule-embedded filtration member), wherein the capsule has a core (content, inner liquid) and a shell, and wherein the core (content) is formed of the composition according to any one of the above [1] to [22] (or wherein the core contains l-menthol and an ingredient (X)).
[25] A tobacco product containing the composition according to any one of the above [1] to [22] (or containing l-menthol and an ingredient (X)).
[26] An inhalation device containing the composition according to any one of the above [1] to [22] (or containing l-menthol and an ingredient (X)).
[27] The inhalation device according to the above [26], wherein the inhalation device is a smoking device.
[28] The tobacco product or inhalation device according to any one of the above [25] to [27], wherein the tobacco product or inhalation device contains the capsule according to the above [23] or the filter according to the above [24].
[29] A perfumery or cosmetic product containing the composition according to any one of the above [1] to [22] (or containing l-menthol and an ingredient (X)).
[30] The perfumery or cosmetic product according to the above [29], wherein the perfumery or cosmetic product is an air freshener, an oral care product, or a cosmetic product.
[31] A food or drink product containing the composition according to any one of the above [1] to [22] (or containing l-menthol and an ingredient (X)).
[32] The food or drink product according to the above [31], wherein the food or drink product is in the form of a capsule.
[33] A pharmaceutical product containing the composition according to any one of the above [1] to [22].
[34] The pharmaceutical product according to the above [33], wherein the pharmaceutical product is a gastric peristalsis inhibitor.
[35] An agent for enhancing dissolution of l-menthol (preventing crystallization of l-menthol), comprising at least one ingredient (X) selected from dicarboxylic acid esters, diol esters, monocarboxylic acid esters, esters of polyols having three or more hydroxy groups, esters of polycarboxylic acids having three or more carboxyl groups, polyol ethers, polyamines, and alcohols having six or more carbon atoms.
[36] An agent for enhancing dissolution of l-menthol, comprising an ingredient (X) satisfying at least one selected from the following requirements (1) and (2):
   (1) the ingredient (X) has a water/octanol partition coefficient (log P) of 10 or less; and
   (2) the ingredient (X) has a hydrophilic-lipophilic balance (HLB) value of 12 or less.
[37] An agent for enhancing dissolution of l-menthol, comprising an ingredient (X) comprising at least one selected from dicarboxylic acid esters, diol esters, monocarboxylic acid esters, esters of polyols having three or more hydroxy groups, esters of polycarboxylic acids having three or more carboxyl groups, polyol ethers, polyamines, and alcohols having six or more carbon atoms, wherein the ingredient (X) satisfies at least one selected from the following requirements (1) and (2) :
   (1) the ingredient (X) has a water/octanol partition coefficient (log P) of 10 or less; and
   (2) the ingredient (X) has a hydrophilic-lipophilic balance (HLB) value of 12 or less.
[38] A method for enhancing dissolution of l-menthol, comprising bringing l-menthol into contact with an ingredient (X) comprising at least one selected from dicarboxylic acid esters, diol esters, monocarboxylic acid esters, esters of polyols having three or more hydroxy groups, esters of polycarboxylic acids having three or more carboxyl groups, polyol ethers, polyamines, and alcohols having six or more carbon atoms.
[39] A method for enhancing dissolution of l-menthol, comprising bringing l-menthol into contact with an ingredient (X) satisfying at least one selected from the following requirements (1) and (2):
   (1) the ingredient (X) has a water/octanol partition coefficient (log P) of 10 or less; and
   (2) the ingredient (X) has a hydrophilic-lipophilic balance (HLB) value of 12 or less.
[40] A method for enhancing dissolution of l-menthol, comprising bringing l-menthol into contact with an ingredient (X) comprising at least one selected from dicarboxylic acid esters, diol esters, monocarboxylic acid esters, esters of polyols having three or more hydroxy groups, esters of polycarboxylic acids having three or more carboxyl groups, polyol ethers, polyamines, and alcohols having six or more carbon atoms, wherein the ingredient (X) satisfies at least one selected from the following requirements (1) and (2):
   (1) the ingredient (X) has a water/octanol partition coefficient (log P) of 10 or less; and
   (2) the ingredient (X) has a hydrophilic-lipophilic balance (HLB) value of 12 or less.
[41] A method for pulmonary l-menthol delivery, comprising using using the capsule according to the above [23] or the filter according to the above [24] (and breaking the capsule).
[42] A method for pulmonary l-menthol delivery, comprising using using the tobacco product, the inhalation device, and/or the perfumery or cosmetic product according to any one of the above [25] to [28].

### [Claim 1]

A composition comprising l-menthol and a solvent, the solvent being a compound which has a water/octanol partition coefficient (log P) of 1.0 to 3.5 and is published in PubChem (PubChem 2019 update: improved access to chemical data. Nucleic acids research, 47(D1), D1102-D1109).

### [Claim 2]

A composition comprising l-menthol and a solvent, the solvent being a compound which has a water/octanol partition coefficient (log P) of 1.2 to 3.5 and is published in PubChem (PubChem 2019 update: improved access to chemical data. Nucleic acids research, 47(D1), D1102-D1109).

### [Claim 3]

A compound comprising l-menthol and a solvent, the solvent being a compound having a hydrophilic-lipophilic balance (HLB) value of 4.2 to 8.6.

### [Claim 4]

A compound comprising l-menthol and a solvent, the solvent being a compound having a hydrophilic-lipophilic balance (HLB) value of 4.2 to 7.5.

### [Claim 5]

The composition according to any one of claims 1 to 4, wherein the amount of the l-menthol is 20 wt% or more relative to the combined amount of the solvent and the l-menthol which is assumed as 100 wt%.

### [Claim 6]

The composition according to any one of claims 1 to 5, wherein the amount of the l-menthol is 35 wt% or more relative to the combined amount of the solvent and the l-menthol which is assumed as 100 wt%.

### [Claim 7]

The composition according to any one of claims 1 to 6, wherein the solvent is a dicarboxylic acid diester.

### [Claim 8]

The composition according to claim 7, wherein the dicarboxylic acid diester is at least one selected from diethyl succinate, diisobutyl adipate, and diethyl sebacate.

### [Claim 9]

The composition according to any one of claims 1 to 6, wherein the solvent is a diol diester.

### [Claim 10]

The composition according to claim 9, wherein the diol diester is at least one of ethylene glycol diacetate, 1,6-hexanediol diacetate, and 1,8-octanediol diacetate.

### [Claim 11]

The composition according to any one of claims 1 to 6, wherein the solvent is a monoester.

### [Claim 12]

The composition according to claim 11, wherein the monoester is at least one selected from ethyl decanoate, ethyl laurate, ethyl palmitate, and benzyl benzoate.

### [Claim 13]

The composition according to any one of claims 1 to 6, wherein the solvent is a triol triester.

### [Claim 14]

The composition according to claim 13, wherein the triol triester is tributyrin.

### [Claim 15]

The composition according to any one of claims 1 to 6, wherein the solvent is a tricarboxylic acid triester.

### [Claim 16]

The composition according to claim 15, wherein the tricarboxylic acid triester is triethyl citrate.

### [Claim 17]

The composition according to any one of claims 1 to 6, wherein the solvent is a diol diether.

### [Claim 18]

The composition according to claim 17, wherein the diol diether is at least one selected from diethylene glycol dibutyl ether and ethylene glycol dibutyl ether.

### [Claim 19]

The composition according to any one of claims 1 to 6, wherein the solvent is an N,N'-alkyldiamine.

### [Claim 20]

The composition according to claim 19, wherein the N,N'-alkyldiamine is N,N'-diethyl-1,6-hexanediamine.

### [Claim 21]

A composition comprising l-menthol and a solvent, the solvent being a mixture of a dicarboxylic acid diester and at least one of C₁₀₋₁₆ alcohols, wherein the amount of the l-menthol is 55 wt% or more relative to the combined amount of the solvent and the l-menthol which is assumed as 100 wt%.

### [Claim 22]

The composition according to claim 21, wherein the amount of the dicarboxylic acid diester is 26 wt% or more and the amount of the C₁₀₋₁₆ alcohol is less than 5 wt% relative to the combined amount of the solvent and the l-menthol which is assumed as 100 wt%.

### [Claim 23]

The composition according to claim 21, wherein the alcohol in the solvent has a water/octanol partition coefficient (log P) of 4.6 to 7.3 and is published in PubChem (PubChem 2019 update: improved access to chemical data. Nucleic acids research, 47 (D1), D1102-D1109).

### [Claim 24]

The composition according to claim 21, wherein the alcohol in the solvent has a hydrophilic-lipophilic balance (HLB) value of 3.1 to 5.0.

### [Claim 25]

The composition according to claim 21 wherein the alcohol in the solvent comprises 1-dodecanol.

### [Claim 26]

The composition according to any one of claims 1 to 6, further comprising a medium chain triglyceride (MCT).

### [Claim 27]

A capsule having the composition according to any one of claims 1 to 26 entrapped in a shell.

### [Claim 28]

A filter for an inhalation device, the filter comprising:
a capsule having at least the composition according to any one of claims 1 to 26 entrapped in a shell, and
a filtration member having the capsule embedded therein.

### [Claim 29]

A tobacco cigarette having the filter for an inhalation device according to claim 28.

### [Claim 30]

An inhalation device having the filter for an inhalation device according to claim 28.

### [Claim 31]

A food or drink product containing the composition according to any one of claims 1 to 26.

### [Claim 32]

A pharmaceutical composition containing the composition according to any one of claims 1 to 26.

### [Claim 33]

A method for dissolving a high concentration of menthol, comprising using the composition according to any one of claims 1 to 6.

### ADVANTAGEOUS EFFECTS OF INVENTION

The present invention provides useful ingredients for dissolving menthol.

Such ingredients, together with menthol, can form a composition (e.g., a solution).

Such ingredients (e.g., solvents) can replace the whole or part of the aforementioned ingredients, such as emulsifiers, d-neomenthol, and MCTs, and achieve dissolution of the menthol.

For this reason, the ingredients according to the present invention are, for example, advantageous for cost reduction and useful for applications where the use of emulsifiers is not desired (e.g., suitable for forming a capsule content).

In particular, some of the ingredients according to the present invention are particularly superior in dissolving menthol (e.g., they are comparable or superior to MCTs in dissolving menthol on an equal mass basis).

For this reason, in another aspect of the present invention, such superior ingredients can be used to efficiently dissolve a higher concentration of menthol (in the composition).

In addition, some of the ingredients according to the present invention do not disturb menthol flavor. For this reason, in another aspect of the present invention, such ingredients can be used to dissolve menthol without disturbing menthol flavor. This particular aspect of the present invention is suitable for applications in which menthol is used for providing its flavor.

As described above, various aspects of the present invention can be provided, and it will be understood that a combination of more than one aspect of the present invention can also be provided. For example, in an aspect of the present invention, l-menthol can be dissolved at a high concentration in an inexpensive solvent while retaining its flavor. This solvent does not disturb encapsulation and thus can be entrapped in seamless capsules.

### DESCRIPTION OF EMBODIMENTS

Hereinafter, the present invention will be described in detail.

The composition of the present invention comprises l-menthol and a specific ingredient, which is referred to as an ingredient (X).

### l-Menthol

The amount (concentration) of the l-menthol in the composition can be selected according to the embodiment of the composition, the type of ingredient (X) to be combined, the application, and other factors, and is not particularly limited. The amount (concentration) of the l-menthol in the composition can usually be selected from a range of about 1 mass% or more (e.g., 3 mass% or more) and is, for example, 5 mass% (or % by weight or wt%) or more (e.g., 10 mass% or more), preferably 15 mass% or more, more preferably 20 mass% or more (e.g., 22 mass% or more), even more preferably 25 mass% or more, particularly preferably 30 mass% or more (e.g., 32 mass% or more); or may be 35 mass% or more (e.g., 38 mass% or more, 40 mass% or more, 42 mass% or more, 45 mass% or more, 48 mass% or more, 50 mass% or more, 52 mass% or more, 55 mass% or more, 58 mass% or more, 60 mass% or more, 62 mass% or more, 65 mass% or more, 68 mass% or more, 70 mass% or more, 72 mass% or more, 75 mass% or more, 78 mass% or more, 80 mass% or more, etc.).

The upper limit of the amount (concentration) of the l-menthol can be selected according to the embodiment of the composition, the type of ingredient (X) (the ability to dissolve the l-menthol), the application, and other factors. For example, the upper limit may be 99 mass%, 97 mass%, 95 mass%, 93 mass%, 90 mass%, 88 mass%, 85 mass%, 82 mass%, 80 mass%, 78 mass%, 75 mass%, 72 mass%, 70 mass%, 68 mass%, 65 mass%, 62 mass%, 60 mass%, 58 mass%, 55 mass%, 52 mass%, 50 mass%, 48 mass%, 45 mass%, 42 mass%, 40 mass%, 38 mass%, 35 mass%, etc.

These upper and lower limits may be combined to set an appropriate range (e.g., 20 to 95 mass%, 30 to 90 mass%, 55 to 85 mass%, etc.) for the amount (concentration) of the l-menthol (the same applies to defining the range described herein).

The amount of the l-menthol may be selected according to the type of ingredients other than the l-menthol (e.g., an ingredient (X), a carrier, a flavoring agent, and others), their combination and ratio, and other factors.

The l-menthol in the composition (e.g., the composition at ordinary temperature or room temperature, or the composition below the melting point of the l-menthol) usually may be dissolved (in a solid solution state) without being solidified (crystallized, purely solidified).

In the composition of the present invention, the l-menthol can be dissolved even at high concentrations as described above (e.g., 20 mass% or more, 25 mass% or more, 30 mass% or more, 35 mass% or more, 40 mass% or more, 50 mass% or more, 60 mass% or more, 65 mass% or more, 70 mass% or more, etc.).

The amount (concentration) of the l-menthol relative to the combined amount of the l-menthol and the ingredient (X) [when the combined amount is assumed as 100 mass% (wt%)] can be selected from the above-described concentration ranges of the l-menthol in the composition (e.g., 20 mass% or more, 30 mass% or more, 50 mass% or more, 20 to 95 mass%, 30 to 90 mass%, 55 to 85 mass%, etc.).

### Ingredient (X)

### Type (1) of ingredient (X)

In the present invention, specific ingredients are used as one type (type (1)) of ingredient (X).

More specifically, the ingredient (X) (the ingredient (X) of type (1)) comprises at least one selected from dicarboxylic acid esters, diol esters, monocarboxylic acid esters, esters of polyols having three or more hydroxy groups, esters of polycarboxylic acids having three or more carboxyl groups, polyol ethers, polyamines, and alcohols having six or more carbon atoms.

The ingredient (X) may be in a liquid or solid form at ordinary temperature or room temperature (e.g., 15 to 35_{°}C).

A solid ingredient (X) in the composition at ordinary temperature or room temperature is often in a liquid form (e.g., dissolved in a liquid ingredient (X) or in an ingredient other than the ingredient (X)).

In particular, the ingredient (X) may at least comprise a liquid ingredient at ordinary temperature or room temperature. Typically, the ingredient (X) (or all the ingredients (X) when two or more kinds of ingredients (X) are used) is in a liquid form at ordinary temperature or room temperature.

The ingredient (X) will be described in detail below.

### Dicarboxylic acid ester

The dicarboxylic acid ester is an ester of a dicarboxylic acid. The dicarboxylic acid ester may be a monoester (half ester) or a diester and is particularly a dicarboxylic acid diester.

The dicarboxylic acid diester is usually an ester of one dicarboxylic acid molecule and two alcohol molecules. The two alcohol molecules may be the same or different molecules. The two alcohol molecules are usually the same molecules.

The dicarboxylic acid is not particularly limited, and examples include aliphatic dicarboxylic acids and aromatic dicarboxylic acids. The aliphatic dicarboxylic acid can be a saturated or unsaturated dicarboxylic acid. The aliphatic dicarboxylic acid may be chained (including branched-chained) or cyclic. The dicarboxylic acid may be, for example, an oxycarboxylic acid.

Specific examples of the dicarboxylic acid include aliphatic dicarboxylic acids [e.g., saturated dicarboxylic acids (e.g., C₂₋₂₀ saturated dicarboxylic acids, preferably C₂₋₁₆ saturated dicarboxylic acids, more preferably C₄₋₁₂ saturated dicarboxylic acids, such as oxalic acid, malonic acid, succinic acid, glutaric acid, adipic acid, pimelic acid, suberic acid, azelaic acid, sebacic acid, and cyclohexane dicarboxylic acid), unsaturated dicarboxylic acids (e.g., C₄₋₂₀ unsaturated dicarboxylic acids, preferably C₂₋₁₆ unsaturated dicarboxylic acids, more preferably C₄₋₁₂ unsaturated dicarboxylic acids, such as fumaric acid, maleic acid, and cyclohexene dicarboxylic acid), etc.] and aromatic dicarboxylic acids [e.g., C₈₋₂₀ aromatic dicarboxylic acids, preferably C₈₋₁₆ aromatic dicarboxylic acids, preferably C₈₋₁₂ aromatic dicarboxylic acids, such as phthalic acid, isophthalic acid, and terephthalic acid].

The alcohol is not particularly limited, and examples include aliphatic alcohols (including aromatic aliphatic alcohols) and aromatic alcohols. The alcohol may be saturated or unsaturated. The alcohol may be chained (including branched-chained) or cyclic. The alcohol may be a monool or a polyol and is usually a monool.

Specific examples of the alcohol (monool) include aliphatic alcohols [e.g., alkanols (e.g., C₁₋₂₀ alkanols, preferably C₁₋₁₂ alkanols, more preferably C₁₋₆ alkanols (e.g., C₁₋₄ alkanols), such as methanol, ethanol, propanol, isopropanol, butanol, isobutanol, tert-butanol, pentanol, hexanol, heptanol, octanol, 2-ethyl hexanol, decanol, dodecanol, and cyclohexanol), aralkyl alcohols (e.g., hydroxy C₁₋₄ alkyl C₆₋₁₀ arenes such as benzyl alcohol and phenethyl alcohol), etc.].

Examples of the dicarboxylic acid ester include esters of all combinations of these dicarboxylic acids and alcohols. Specific examples of the dicarboxylic acid diester include aliphatic dicarboxylic acid diesters {e.g., saturated dicarboxylic acid diesters [e.g., diesters (e.g., diesters with aliphatic alcohols, such as dialkyl esters (e.g., C₁₋₁₀ alkyl esters, C₁₋₆ alkyl esters, etc.)) of C₂₋₂₀ saturated dicarboxylic acids, such as diethyl malonate, dimethyl succinate, diethyl succinate, diethyl glutarate, diisobutyl adipate, dipropyl adipate, diethyl pimelate, diethyl suberate, diethyl azelate, and diethyl sebacate], unsaturated dicarboxylic acid diesters [e.g., diesters (e.g., diesters with aliphatic alcohols) of C₄₋₂₀ unsaturated dicarboxylic acids, such as diethyl fumarate], etc.} and aromatic dicarboxylic acid esters [e.g., esters (e.g., diesters with aliphatic alcohols, such as dialkyl esters) of C₈₋₂₀ aromatic dicarboxylic acids, such as diethyl phthalate] .

Among these dicarboxylic acid esters, aliphatic dicarboxylic acid diesters are particularly desirable, and from the perspective of odor and others, diesters of saturated or unsaturated dicarboxylic acids having four or more carbon atoms (e.g., diesters of C₄₋₂₀ saturated or unsaturated dicarboxylic acids, such as diethyl succinate, diethyl sebacate, diisobutyl adipate, and diethyl fumarate) are desirable.

A single kind of dicarboxylic acid diester or a combination of two or more kinds of dicarboxylic acid diesters may be used.

### Diol ester

The diol ester is an ester of a diol. The diol ester may be a monoester or diester and is particularly a diol diester.

The diol diester is usually an ester of one diol molecule and two carboxylic acid molecules. The two carboxylic acid molecules may be the same or different molecules. The two carboxylic acid molecules are usually the same molecules.

The diol is not particularly limited, and examples include aliphatic diols (including aromatic aliphatic diols) and aromatic diols. The aliphatic diol may be saturated or unsaturated. The aliphatic diol may be chained (including branched-chained) or cyclic.

Specific examples of the diol include aliphatic diols [e.g., alkanediols (e.g., C₂₋₂₀ alkanediols, preferably C₂₋₁₆ alkanediols, more preferably C₂₋₁₂ alcohols, such as ethylene glycol, 1,3-propanediol, propylene glycol, 1,4-butanediol, butylene glycol, pentanediol, hexanediol, heptanediol, octanediol, decanediol, dodecanediol, cyclohexanediol, and cyclohexanedimethanol), polyalkanediols (e.g., di- to hexa-C₂₋₆ alkanediols such as diethylene glycol, dipropylene glycol, and triethylene glycol), hydroxyalkylarenes (e.g., di(hydroxy C₁₋₄ alkyl) C₆₋₁₀ arenes such as xylylene glycol), etc.].

The carboxylic acid is not particularly limited, and examples include aliphatic carboxylic acids (including aromatic aliphatic carboxylic acids) and aromatic carboxylic acids. The carboxylic acid may be saturated or unsaturated. The carboxylic acid may be chained (including branched-chained) or cyclic. The carboxylic acid may be, for example, an oxycarboxylic acid.

The carboxylic acid may be a monocarboxylic acid or a polycarboxylic acid and is typically a monocarboxylic acid.

Specific examples of the carboxylic acid include monocarboxylic acids including aliphatic carboxylic acids (e.g., C₁₋₃₀ aliphatic carboxylic acids, preferably C₁₋₁₂ aliphatic carboxylic acids, more preferably C₁₋₆ aliphatic carboxylic acids, such as formic acid, acetic acid, propionic acid, butyric acid, valeric acid, isovaleric acid, caproic acid, enanthic acid, caprylic acid, pelargonic acid, capric acid, lauric acid, myristic acid, palmitic acid, margaric acid, stearic acid, oleic acid, and cyclohexane carboxylic acid) and aromatic carboxylic acids (e.g., carboxy C₆₋₁₀ arenes such as benzoic acid and salicylic acid).

Examples of the diol ester include esters of all combinations of these diols and carboxylic acids.

Specific examples of the diol ester include diol diesters including aliphatic diol diesters [e.g., diesters (e.g., diesters with aliphatic carboxylic acids) of C₂₋₂₀ aliphatic diols, such as ethylene glycol diacetate, propylene glycol diacetate, 1,4-butanediol diacetate, butylene glycol diacetate, 1,6-hexanediol diacetate, 1,8-octanediol diacetate, ethylene glycol bis(butyrate), and triethylene glycol dibutylate].

Particularly preferred are diesters (e.g., diesters with aliphatic carboxylic acids) of aliphatic diols having three or more carbon atoms (e.g., C₃₋₂₀ aliphatic diols, C₄₋₁₆ aliphatic diols, C₆₋₁₂ aliphatic diols, etc.) from the perspective of the dissolution of the l-menthol and others.

A single kind of diol ester or a combination of two or more kinds of diol esters may be used.

### Monocarboxylic acid ester

The monocarboxylic acid ester (monocarboxylic acid monoester) is an ester of a monocarboxylic acid (an ester of a monocarboxylic acid and an alcohol, that is, an ester of one alcohol molecule and one carboxylic acid molecule). The alcohol may be a monool or a polyol and is usually a monool.

The monocarboxylic acid is not particularly limited, and examples include aliphatic carboxylic acids (including aromatic aliphatic carboxylic acids) and aromatic carboxylic acids. The carboxylic acid may be saturated or unsaturated. The carboxylic acid may be chained (including branched-chained) or cyclic. The carboxylic acid may be, for example, an oxycarboxylic acid.

Specific examples of the monocarboxylic acid include aliphatic carboxylic acids [e.g., C₁₋₃₀ aliphatic carboxylic acids, preferably C₄₋₂₈ aliphatic carboxylic acids, more preferably C₆₋₂₄ aliphatic carboxylic acids (e.g., aliphatic carboxylic acids having 14 carbon atoms or less), such as formic acid, acetic acid, propionic acid, butyric acid, valeric acid, isovaleric acid, caproic acid, enanthic acid, caprylic acid, pelargonic acid, capric acid, lauric acid, myristic acid, palmitic acid, margaric acid, stearic acid, oleic acid, linoleic acid, linolenic acid, and cyclohexane carboxylic acid] and aromatic carboxylic acids (e.g., carboxy C₆₋₁₀ arenes such as benzoic acid and salicylic acid).

The alcohol is not particularly limited, and examples include aliphatic alcohols (including aromatic aliphatic alcohols) and aromatic alcohols. The alcohol may be saturated or unsaturated. The alcohol may be chained (including branched-chained) or cyclic.

Specific examples of the alcohol (monool) include aliphatic alcohols [e.g., alkanols (e.g., C₁₋₂₀ alkanols, preferably C₁₋₁₂ alkanols, more preferably C₁₋₆ alkanols (e.g., C₁₋₄ alkanols), such as methanol, ethanol, propanol, isopropanol, butanol, isobutanol, tert-butanol, pentanol, hexanol, heptanol, octanol, 2-ethyl hexanol, decanol, dodecanol, and cyclohexanol), aralkyl alcohols (e.g., hydroxy C₁₋₄ alkyl C₆₋₁₀ arenes such as benzyl alcohol and phenethyl alcohol), etc.].

Examples of the monocarboxylic acid ester include esters of all combinations of these monocarboxylic acids and alcohols.

Specific examples of the monocarboxylic acid ester include aliphatic carboxylic acid esters [aliphatic monocarboxylic acid esters (monoesters), for example, esters (e.g., esters with aliphatic alcohols) of C₁₋₃₀ aliphatic carboxylic acids (e.g., C₄₋₂₈ aliphatic carboxylic acids, C₆₋₂₄ aliphatic carboxylic acids, etc.), such as ethyl decanoate, ethyl laurate, ethyl palmitate, and ethyl palmitate] and aromatic carboxylic acid esters [aromatic monocarboxylic acid esters (monoesters), for example, esters (e.g., diesters with aliphatic alcohols) of C₇₋₂₀ aromatic carboxylic acids, such as benzyl benzoate and benzyl salicylate].

A single kind of monocarboxylic acid ester or a combination of two or more kinds of monocarboxylic acid esters may be used.

### Ester of polyol having three or more hydroxy groups

The ester of a polyol having three or more hydroxy groups may be a partial ester (e.g., monoester or diester) or a complete ester (e.g., triester when the alcohol is a triol) and is typically a complete ester.

In the case where the ester of a polyol having three or more hydroxy groups is an ester of two or more carboxylic acid molecules, the two or more carboxylic acid molecules may be the same or different molecules. The two or more carboxylic acid molecules are typically the same molecules.

The polyol having three or more hydroxy groups is not particularly limited, and examples include aliphatic polyols (including aromatic aliphatic polyols) and aromatic polyols. The polyol (aliphatic polyol) having three or more hydroxy groups may be saturated or unsaturated. The polyol (aliphatic polyol) having three or more hydroxy groups may be chained (including branched-chained) or cyclic.

The number of hydroxy groups in the polyol having three or more hydroxy groups is 3 or more and is, for example, 3 to 10, preferably 3 to 6, and more preferably 3 to 5 (e.g., 3).

Specific examples of the polyol having three or more hydroxy groups include aliphatic polyols {e.g., alkane tri- to hexa-ols [e.g., alkanetriols (e.g., C₃₋₁₀ alkanetriols such as glycerol, 1,2,4-butanetriol, trimethylolpropane, and trimethylolpropane), alkanetetraols (e.g., C₃₋₁₀ alkanetetraols such as erythritol and pentaerythritol), etc.], polyalkane polyols [e.g., poly(alkane tri- or hexa-ols) such as diglycerol, ditrimethylolpropane, and dipentaerythritol], etc.}.

The carboxylic acid is not particularly limited, and examples include aliphatic carboxylic acids (including aromatic aliphatic carboxylic acids) and aromatic carboxylic acids. The carboxylic acid may be saturated or unsaturated. The carboxylic acid may be chained (including branched-chained) or cyclic. The carboxylic acid may be, for example, an oxycarboxylic acid.

The carboxylic acid may be a monocarboxylic acid or a polycarboxylic acid and is typically a monocarboxylic acid.

Specific examples of the carboxylic acid (monocarboxylic acid) include aliphatic carboxylic acids (e.g., C₁₋₃₀ aliphatic carboxylic acids, preferably C₁₋₁₂ aliphatic carboxylic acids, more preferably C₁₋₆ aliphatic carboxylic acids, such as formic acid, acetic acid, propionic acid, butyric acid, valeric acid, isovaleric acid, caproic acid, enanthic acid, caprylic acid, pelargonic acid, capric acid, lauric acid, myristic acid, palmitic acid, margaric acid, stearic acid, oleic acid, and cyclohexane carboxylic acid) and aromatic carboxylic acids (e.g., carboxy C₆₋₁₀ arenes such as benzoic acid and salicylic acid).

Examples of the ester of a polyol having three or more hydroxy groups include esters of all combinations of these carboxylic acids and polyols.

Specific examples of the ester of a polyol having three or more hydroxy groups include triol esters {e.g., triol triesters [e.g., triesters of glycerol and aliphatic carboxylic acids (fatty acids) (e.g., triesters of glycerol and fatty acids having six or less carbon atoms (e.g., five or less carbon atoms, four or less carbon atoms, etc.), such as tri-C₁₋₆ fatty acid esters, tri-C₁₋₅ fatty acid esters, and tri-C₁₋₄ fatty acid esters), such as tributyrin], etc.}.

A single kind of ester of a polyol having three or more hydroxy groups or a combination of two or more kinds of esters of a polyol having three or more hydroxy groups may be used.

### Ester of polycarboxylic acid having three or more carboxyl groups

The ester of a polycarboxylic acid having three or more carboxyl groups may be a partial ester (e.g., monoester or diester) or a complete ester (e.g., triester when the polycarboxylic acid is a tricarboxylic acid) and is typically a complete ester.

In the case where the ester of a polycarboxylic acid having three or more carboxyl groups is an ester of two or more alcohol molecules, the two or more alcohol molecules may be the same or different molecules. The two or more alcohol molecules are typically the same molecules.

The polycarboxylic acid having three or more carboxyl groups is not particularly limited, and examples include aliphatic polycarboxylic acids (including aromatic aliphatic polycarboxylic acids). The polycarboxylic acid having three or more carboxyl groups may be saturated, unsaturated, or aromatic. The polycarboxylic acid having three or more carboxyl groups may be chained (including branched-chained) or cyclic.

The number of carboxyl groups in the polycarboxylic acid having three or more carboxyl groups is 3 or more and is, for example, 3 to 10, preferably 3 to 6, and more preferably 3 to 5 (e.g., 3).

Specific examples of the polycarboxylic acid having three or more carboxyl groups include aliphatic polycarboxylic acids {e.g., aliphatic tri- to hexa-carboxylic acids [e.g., poly (aliphatic tri- to hexa-carboxylic acids) such as aliphatic tricarboxylic acids (e.g., C₅₋₁₂ aliphatic tricarboxylic acids such as citric acid and aconitic acid)], etc.}.

The alcohol is not particularly limited, and examples include aliphatic alcohols (including aromatic aliphatic alcohols) and aromatic alcohols. The alcohol may be saturated or unsaturated. The alcohol may be chained (including branched-chained) or cyclic. The alcohol may be a monool or a polyol and is usually a monool.

Specific examples of the alcohol (monool) include aliphatic alcohols [e.g., alkanols (e.g., C₁₋₂₀ alkanols, preferably C₁₋₁₂ alkanols, more preferably C₁₋₆ alkanols (e.g., C₁₋₄ alkanols), such as methanol, ethanol, propanol, isopropanol, butanol, isobutanol, tert-butanol, pentanol, hexanol, heptanol, octanol, 2-ethyl hexanol, decanol, dodecanol, and cyclohexanol), aralkyl alcohols (e.g., hydroxy C₁₋₄ alkyl C₆₋₁₀ arenes such as benzyl alcohol and phenethyl alcohol), etc.].

Examples of the ester of a polycarboxylic acid having three or more carboxyl groups include esters of all combinations of these polycarboxylic acids and alcohols.

Specific examples of the ester of a polycarboxylic acid having three or more carboxyl groups include tricarboxylic acid esters {e.g., triesters of citric acid [e.g., triesters of citric acid and aliphatic alcohols (e.g., tri-C₁₋₆ alkyl citrates such as triethyl citrate), etc.], etc.}.

### Polyol ether

The polyol ether may be a partial ether (e.g., monoether) or a complete ether (e.g., diether when the polyalcohol is a diol) and is typically a complete ether.

In the case where the polyol ether is an ether of two or more alcohol molecules, the two or more alcohol molecules may be the same or different molecules. The two or more alcohol molecules are typically the same molecules.

The polyol is not particularly limited, and examples include aliphatic polyols (including aromatic aliphatic polyols) and aromatic polyols. The aliphatic polyol may be saturated or unsaturated. The aliphatic polyol may be chained (including branched-chained) or cyclic.

The number of hydroxy groups in the polyol is 2 or more and is, for example, 2 to 10, preferably 2 to 6, and more preferably 2 to 4 (e.g., 2).

Specific examples of the polyol include diols and polyols having three or more hydroxy groups.

Examples of the diol include aliphatic diols [e.g., alkanediols (e.g., C₂₋₂₀ alkanediols, preferably C₂₋₁₆ alkanediols, more preferably C₂₋₁₂ alcohols, such as ethylene glycol, 1,3-propanediol, propylene glycol, 1,4-butanediol, butylene glycol, pentanediol, hexanediol, heptanediol, octanediol, decanediol, dodecanediol, cyclohexanediol, and cyclohexanedimethanol), polyalkanediols (e.g., di- to hexa-C₂₋₆ alkanediols such as diethylene glycol, dipropylene glycol, and triethylene glycol), dihydroxyalkylarenes (e.g., di(hydroxy C₁₋₄ alkyl) C₆₋₁₀ arenes such as xylylene glycol), etc.].

Examples of the polyol having three or more hydroxy groups include aliphatic polyols {e.g., alkane tri- to hexa-ols [e.g., alkanetriols (e.g., C₃₋₁₀ alkanetriols such as glycerol, 1,2,4-butanetriol, trimethylolpropane, and trimethylolpropane), alkanetetraols (e.g., C₃₋₁₀ alkanetetraols such as erythritol and pentaerythritol), etc.], polyalkane polyols [e.g., poly(alkane tri- or hexa-ols) such as diglycerol, ditrimethylolpropane, and dipentaerythritol], etc.}.

The alcohol forming the ether group is not particularly limited, and examples include aliphatic alcohols (including aromatic aliphatic alcohols) and aromatic alcohols. The alcohol may be saturated or unsaturated. The alcohol may be chained (including branched-chained) or cyclic. The alcohol may be a monool or a polyol and is usually a monool.

Specific examples of the alcohol (monool) include aliphatic alcohols [e.g., alkanols (e.g., C₁₋₂₀ alkanols, preferably C₁₋₁₂ alkanols, more preferably C₁₋₆ alkanols (e.g., C₁₋₄ alkanols), such as methanol, ethanol, propanol, isopropanol, butanol, isobutanol, tert-butanol, pentanol, hexanol, heptanol, octanol, 2-ethyl hexanol, decanol, dodecanol, and cyclohexanol), aralkyl alcohols (e.g., hydroxy C₁₋₄ alkyl C₆₋₁₀ arenes such as benzyl alcohol and phenethyl alcohol), etc.].

Examples of the polyol ether include ethers of all combinations of these diols, polyols, and alcohols.

Specific examples of the polyol ether include aliphatic polyol ethers {e.g., aliphatic diol ethers [e.g., ethers (e.g., mono- or di-ethers with aliphatic alcohols, such as alkyl ethers (e.g., C₁₋₁₀ alkyl ethers, C₁₋₆ alkyl ethers, etc.)) of C₂₋₂₀ aliphatic diols, such as 1,2-diethoxyethane, ethylene glycol dibutyl ether, diethylene glycol diethyl ether, and diethylene glycol dibutyl ether], etc.}.

A single kind of polyol ether or a combination of two or more kinds of polyol ethers may be used.

### Polyamine

The number of amino groups (nitrogen atoms) in the polyamine is 2 or more and is, for example, 2 to 10, preferably 2 to 6, and more preferably 2 to 4 (e.g., 2).

The polyamine is not particularly limited and may be an aliphatic polyamine (including an aromatic aliphatic polyamine) or an aromatic polyamine. The aliphatic polyamine may be saturated or unsaturated. The aliphatic polyamine may be chained (including branched-chained) or cyclic.

The amino groups may be substituted amino groups. Examples of the substituent in the substituted amino group include hydrocarbon groups [e.g., alkyl groups (e.g., C₁₋₁₀ alkyl groups such as a methyl group, an ethyl group, a propyl group, an isopropyl group, a butyl group, an isobutyl group, a tert-butyl group, a hexyl group, and a 2-ethylhexyl group), etc.].

In the substituted amino group, one or two hydrogen atoms of the two hydrogen atoms forming the amino group are substituted.

Specific examples of the polyamine include polyamines (N-unsubstituted polyamines) [e.g., diamines (e.g., C₁₋₁₀ alkanediamines such as ethylenediamine, putrescine, cadaverine, and 1,6-hexanediamine)] and N-substituted polyamines [e.g., N-substituted diamines (e.g., N-mono- to tetra-alkyl (e.g., C₁₋₄ alkyl) C₁₋₁₀ alkanediamines such as N,N'-diethyl-1,6-hexanediamine)].

A single kind of polyamine or a combination of two or more kinds of polyamines may be used.

### Alcohol having six or more carbon atoms

The alcohol having six or more carbon atoms is not particularly limited, and examples include aliphatic alcohols (including aromatic aliphatic alcohols) and aromatic alcohols. Typically, the alcohol is an aliphatic alcohol.

The aliphatic alcohol may be saturated or unsaturated. The aliphatic alcohol may be chained (including branched-chained) or cyclic and is typically chained.

The alcohol having six or more carbon atoms may be a monool or a polyol and is usually a monool.

The alcohol having six or more carbon atoms may be a primary, secondary, or tertiary alcohol and is typically a primary alcohol.

Specific examples of the alcohol (monool) having six or more carbon atoms include aliphatic alcohols [e.g., alkanols (e.g., C₆₋₃₀ alkanols, preferably C₈₋₁₈ alkanols, more preferably C₁₀₋₁₆ alkanols, such as hexanol, heptanol, octanol, 2-ethyl hexanol, nonanol, decanol, undecanol, dodecanol, tridecanol, tetradecanol, pentadecanol, and hexadecanol), aralkyl alcohols (e.g., hydroxy C₁₋₄ alkyl C₆₋₁₀ arenes such as benzyl alcohol and phenethyl alcohol), etc.].

Typical examples of the alcohol having six or more carbon atoms include C₁₀₋₁₆ alcohols. C₁₀₋₁₆ alcohols are alcohols (e.g., straight-chained primary alcohols) having 10 to 16 carbon atoms.

Examples of the alcohol having six or more carbon atoms include C₁₀₋₁₆ alcohols [e.g., alkanols (in particular, straight-chained primary alcohols) such as 1-decanol, 1-undecanol, 1-dodecanol, 1-tridecanol, 1-tetradecanol, 1-pentadecanol, and 1-hexadecanol] and aralkyl alcohols (e.g., benzyl alcohol). 1-Decanol, benzyl alcohol, etc., are highly capable of dissolving menthol, but may have a slightly unusual odor as compared with other alcohols having six or more carbon atoms, although it depends on their amount contained in the composition. On the other hand, 1-tridecanol, 1-tetradecanol, 1-pentadecanol, and 1-hexadecanol have no odor, but are less capable of dissolving menthol than alcohols having a fewer carbon atoms, although it depends on their amount contained in the composition.

In the case of using alcohols having six or more carbon atoms, these issues may be taken into consideration to select an appropriate alcohol according to the desired application and other factors . For example, in the case of using benzyl alcohol, limiting its amount is desirable from the perspective of odor, and on top of that, combining with another ingredient (X) (e.g., a dicarboxylic acid diester) or another ingredient (e.g., an MCT) can achieve a balance between the dissolution of the menthol and the retention of menthol flavor. On the other hand, 1-dodecanol and others have no odor and are satisfactory in terms of the dissolution of the menthol, as compared to other alcohols having six or more carbon atoms, and are therefore particularly desirable.

A single kind of alcohol having six or more carbon atoms or a combination of two or more kinds of alcohols having six or more carbon atoms may be used.

In addition, the alcohol having six or more carbon atoms (e.g., C₁₀₋₁₆ alcohols) may be used in combination with other ingredients [such as MCTs, ingredients other than the alcohol having six or more carbon atoms (including the ingredient (X) of type (2)), which are described later] . Such a combined use with other ingredients can provide a composition that advantageously achieves a balance between the dissolution of the menthol and the retention of menthol flavor.

In the case where the alcohol having six or more carbon atoms is used in combination with the ingredient (X) other than the alcohol having six or more carbon atoms (e.g., a dicarboxylic acid ester), the amounts of these ingredients can be selected according to the desired dissolution of the menthol, retention of menthol flavor, and other factors. For example, the amount of the alcohol having six or more carbon atoms relative to the combined amount of the alcohol having six or more carbon atoms and the ingredient (X) other than the alcohol having six or more carbon atoms may be 1 to 99 mass% (e.g., 2 to 98 mass%) and is preferably 5 to 95 mass% (e.g., 10 to 90 mass%). This amount may be 5 mass% or more, 10 mass% or more, 15 mass% or more, 20 mass% or more, 30 mass% or more, 40 mass% or more, 50 mass% or more, 95 mass% or less, 90 mass% or less, 85 mass% or less, 80 mass% or less, 75 mass% or less, 70 mass% or less, 60 mass% or less, 50 mass% or less, etc.

### Type (2) of ingredient (X)

In the present invention, ingredients identified based on specific indices are used as another type (type (2)) of ingredient (X).

More specifically, the ingredient (X) (the ingredient (X) of type (2)) may be an ingredient satisfying at least one selected from the following requirements (1) and (2):
(1) the ingredient (X) has a water/octanol partition coefficient (log P) of 10 or less; and
(2) the ingredient (X) has a hydrophilic-lipophilic balance (HLB) value of 12 or less.

The use of such indices allows efficient identification (selection) of the ingredient (X) useful for dissolving (preventing crystallization of) the l-menthol.

The ingredient (X) has only to satisfy at least one of the requirements (1) and (2), but may satisfy both requirements.

The ingredient (X) preferably satisfies the requirement (1), but may satisfy both the requirements (1) and (2).

The ingredient (X) of type (1) may or may not satisfy the above requirements. In other words, the ingredient (X) of type (1) does not necessarily have to satisfy the above requirements because this type of ingredient (X) is useful for dissolving the l-menthol in the composition. However, selecting ingredients satisfying the above requirements from the ingredients exemplifying the ingredient (X) of type (1) is a more efficient way for selecting ingredients useful for dissolving the l-menthol.

Each requirement is described in detail below.

### Requirement (1): Water/octanol partition coefficient (log P)

The water/octanol partition coefficient (log P) is a coefficient for a compound distributed in a water-octanol solvent system, representing the concentration ratio of the compound between the two phases separated after mixing equal amounts of water and octanol with the compound.

log P = X means that when the amount dissolved in the water phase is 1, the amount dissolved in the octanol phase is 10^{X}.

The log P values and the log P values (calculated values) of some compounds (individual compounds) are published in PubChem (e.g., Non Patent Literature 1 and Non Patent Literature 2).

### Non Patent Literature 1:

Kim, S., Chen, J., Cheng, T., Gindulyte, A., He, J., He, S., Li, Q., Shoemaker, B. A., Thiessen, P. A., Yu, B., Zaslavsky, L., Zhang, J., & Bolton, E. E. (2019). PubChem 2019 update: improved access to chemical data. Nucleic acids research, 47(D1), D1102-D1109.

### Non Patent Literature 2:

T. Cheng, Y. Zhao, X. Li, F. Lin, Y. Xu, X. Zhang, Y. Li, R. Wang, L. Lai. Computation of octanol-water partition coefficients by guiding an additive model with knowledge. J. Chem. Inf. Model., 47(6):2140-2148, 2007.

The log P values of some of the dicarboxylic acid diesters, diol diesters, monoesters, diol diethers, N,N'-alkyldiamines, and alcohols having six or more carbon atoms, and other compounds shown above as examples of the ingredient (X) of type (1) are shown in Table 1 below.

### [Table 1]

**Table 1 Log P values of dicarboxylic acid diesters, diol diesters, monoesters, diol diethers, N,N'-alkyldiamines, and alcohols having six or more carbon atoms.**

| Compound group | Compound name | Log P value |
|---|---|---|
| Dicarboxylic acid diester | Diethyl malonate | 1.0 |
| | Diethyl succinate | 1.2 |
| | Diisobutyl adipate | 3.2 |
| | Diethyl glutarate | 1.0 |
| | Diethyl pimelate | 1.9 |
| | Diethyl suberate | 2.4 |
| | Diethyl azelate | 2.9 |
| | Diethyl sebacate | 3.5 |
| | Diethyl phthalate | 2.5 |
| | Diethyl fumarate | 0.9 |
| Diol diester | Ethylene glycol diacetate | 0.0 |
| | 1,2-Propylene glycol diacetate | 0.7 |
| | 1,4-Butanediol diacetate | 0.7 |
| | 1,3-Butylene glycol diacetate | 0.8 |
| | 1,6-Hexanediol diacetate | 1.4 |
| | 1,8-Octanediol diacetate | 2.5 |
| | Propylene glycol dibutyrate | 2.1 |
| Monoester | Benzyl benzoate | 4.0 |
| | Benzyl salicylate | 3.2 |
| | Ethyl decanoate | 4.6 |
| | Ethyl laurate | 5.6 |
| | Ethyl palmitate | 7.8 |
| Triol Triester | Tributyrin | 2.4 |
| Tricarboxylic acid triester | Triethyl citrate | 0.1 |
| Diol diether | Diethylene glycol dibutyl ether | 1.9 |
| | Diethylene glycol diethyl ether | 0.4 |
| | 1,2-Diethoxyethane | 0.7 |
| | Ethylene glycol dibutyl ether | 2.5 |
| N,N'-alkyldiamine | N,N'-diethyl-1,6-hexanediamine | 1.5 |
| Alcohol having six or more carbon atoms | 1-Decanol | 4.6 |
| | 1-Dodecanol | 5.1 |
| | 1-Hexadecanol | 7.3 |
| | Benzyl alcohol | 1.1 |

For the requirement (1), the log P value is set at 10 or less. For example, the log P value is 9.5 or less (e.g., 9 or less), preferably 8.5 or less (e.g., 8.2 or less), more preferably 8 or less (e.g., 7.5 or less, 7.3 or less); or may be 7 or less, 6 or less, 5 or less, 4 or less, 3.5 or less, etc.

The lower limit of the log P value is not particularly specified, and may be 0 (the limit of calculation, measurement, or detection) or a finite value.

More specifically, the lower limit of the log P value (a finite log P value) is, for example, 0.1, 0.2, 0.3, 0.4, 0.5, 0.6, 0.7, 0.8, 0.9, 1, 1.1, 1.2, etc. The log P value is preferably 0.9 or more (e.g., 1 or more, 1.1 or more, 1.2 or more, etc.) or may be 2 or more (e.g., 2.5 or more, 3 or more, 3.5 or more, 4 or more, 4.5 or more, or 4.6 or more).

The range of the log P value may be set similarly as described above, for example, by combining any two of the values listed in Table 1 above or by combining the above-described upper and lower limits as appropriate (e.g., 0.1 to 10, 0.5 to 7.3, 1 to 3.5, 1.2 to 3.5, 4.6 to 7.3, etc.).

The ingredients (chemical species, compound groups) that satisfy the requirement (1) are not limited to, but can include the chemical species which exemplify the ingredient (X) of type (1) as listed in Table 1 above (e.g., dicarboxylic acid esters, diol esters, monocarboxylic acid esters, esters of polyols having three or more hydroxy groups, esters of polycarboxylic acids having three or more carboxyl groups, polyol ethers, polyamines, and alcohols having six or more carbon atoms).

### Requirement (2): Hydrophilic-lipophilic balance (HLB)

Hydrophilic-lipophilic balance (HLB) is a measure indicating whether an ingredient (molecule) is aqueous (inorganic) or oily (organic), and the HLB value is calculated based on the molecular structure of the ingredient (e.g., Non Patent Literature 3 and 4).

### Non Patent Literature 3:

Systematic characterization of organic compounds (for mixtures) (written by Atsushi Fujita and Masami Akatsuka, published by Kazamashobo, 1974)

### Non Patent Literature 4:

Organic conceptual diagram -basics and applications- (written by Yoshio Kohda, published by SANKYO SHUPPAN, 1984)

The organic and inorganic values are specific for individual functional groups and other components in a molecule. The organic and inorganic values of the whole molecule can be calculated by adding the corresponding values together. For example, the organic and inorganic values for individual functional groups and other components are listed in Table 2 below.

### [Table 2]

**Table 2 Examples of organic and inorganic values based on functional groups**

| Functional groups, etc. | Organic value | Inorganic value |
|---|---|---|
| Carbon atom (single) | 20 | 0 |
| Amide | 0 | 200 |
| Carboxylic acid | 0 | 150 |
| Lactone | 0 | 120 |
| Alcohol | 0 | 100 |
| Amine | 0 | 70 |
| Ketone | 0 | 65 |
| Ester | 0 | 80 |
| Ether | 0 | 20 |
| Benzene ring | 0 | 15 |
| Ring | 0 | 10 |
| Triple bond | 0 | 3 |
| Double bond | 0 | 2 |
| Iso branch | -10 | 0 |
| Tert branch | -20 | 0 |
| Quaternary carbon | 20 | 0 |

Based on the organic and inorganic values of the whole molecule, the HLB value can be calculated by the following formula: inorganic value/organic value × 10. Table 3 below show the organic and inorganic values and HLB values of some of the compounds exemplifying the ingredient (X) of type (1), including dicarboxylic acid diesters, diol diesters, monoesters, diol diethers, N,N'-alkyldiamines, and alcohols having six or more carbon atoms.

### [Table 3]

**Table 3 Organic and inorganic values and HLB values of dicarboxylic acid diesters, diol diesters, monoesters, diol diethers, N,N'-alkyldiamines, and alcohols having six or more carbon atoms.**

| Compound group | Compound name | Organic value | Inorganic value | HLB value |
|---|---|---|---|---|
| Dicarboxylic acid diester | Diethyl malonate | 140 | 120 | 8.57 |
| | Diethyl succinate | 160 | 120 | 7.50 |
| | Diisobutyl adipate | 200 | 120 | 6.00 |
| | Diethyl glutarate | 180 | 120 | 6.67 |
| | Diethyl pimelate | 220 | 120 | 5.45 |
| | Diethyl suberate | 240 | 120 | 5.00 |
| | Diethyl azelate | 260 | 120 | 4.62 |
| | Diethyl sebacate | 280 | 120 | 4.29 |
| | Diethyl phthalate | 240 | 135 | 5.63 |
| | Diethyl fumarate | 160 | 122 | 7.63 |
| Diol diester | Ethylene glycol diacetate | 120 | 120 | 10.00 |
| | 1,2-Propylene glycol diacetate | 140 | 120 | 8.57 |
| | 1,4-Butanediol diacetate | 160 | 120 | 7.50 |
| | 1,3-Butylene glycol diacetate | 160 | 120 | 7.50 |
| | 1,6-Hexanediol diacetate | 200 | 120 | 6.00 |
| | 1,8-Octanediol diacetate | 240 | 120 | 5.00 |
| | Propylene glycol dibutyrate | 220 | 120 | 5.45 |
| Monoester | Benzyl benzoate | 280 | 90 | 3.21 |
| | Benzyl salicylate | 280 | 190 | 6.79 |
| | Ethyl decanoate | 240 | 60 | 2.50 |
| | Ethyl laurate | 280 | 60 | 2.14 |
| | Ethyl palmitate | 360 | 60 | 1.67 |
| Triol Triester | Tributyrin | 300 | 180 | 6.00 |
| Tricarboxylic acid triester | Triethyl citrate | 240 | 280 | 11.67 |
| Diol diether | Diethylene glycol dibutyl ether | 240 | 60 | 2.50 |
| | Diethylene glycol diethyl ether | 160 | 60 | 3.75 |
| | 1,2-Diethoxyethane | 120 | 40 | 3.33 |
| | Ethylene glycol dibutyl ether | 200 | 40 | 2.00 |
| N,N'-alkyldiamine | N,N'-diethyl-1,6-hexanediamine | 200 | 140 | 7.00 |
| Alcohol having six or more carbon atoms | 1-Decanol | 200 | 100 | 5.00 |
| | 1-Dodecanol | 240 | 100 | 4.17 |
| | 1-Hexadecanol | 320 | 100 | 3.13 |
| | Benzyl alcohol | 140 | 115 | 8.21 |

For the requirement (2), the HLB value is set at 12 or less (e.g., 11.8 or less, or 11.5 or less). For example, the HLB value is 11 or less (e.g., 10.5 or less), preferably 10 or less (e.g., 9.5 or less), more preferably 9.2 or less; or may be 9 or less, 8.8 or less, 8.6 or less, 8.4 or less, 8.2 or less, 8 or less, 7.9 or less, 7.8 or less, 7.6 or less, 7.5 or less, 7.2 or less, 7 or less, 6.5 or less, 6 or less, 5.5 or less, 5.2 or less, 5 or less, etc.

The lower limit of the HLB value is not particularly specified and is, for example, 0.1, 0.2, 0.3, 0.4, 0.5, 0.6, 0.7, 0.8, 0.9, 1, 1.1, 1.2, 1.3, 1.4, etc. The HLB value is preferably 1.5 or more (e.g., 1.6 or more, 1.8 or more, 2 or more, 2.2 or more, 2.5 or more, 2.8 or more, etc.) or may be 3 or more (e.g., 3.1 or more, 3.2 or more, 3.5 or more, 3.6 or more, 3.7 or more, 3.8 or more, 4 or more, or 4.2 or more).

More specifically, the range of the HLB value may be set similarly as described above, for example, by combining any two of the values listed in Table 3 above or by combining the above-described upper and lower limits as appropriate (e.g., 0.1 to 12, 1.67 to 8.57, 3.33 to 8.2, 4.2 to 8.6, 4.2 to 7.5, 3.1 to 5, etc.).

The ingredients (chemical species, compound groups) that satisfy the requirement (2) are not limited to, but can include the chemical species which exemplify the ingredient (X) of type (1) as listed in Table 3 above (e.g., dicarboxylic acid esters, diol esters, monocarboxylic acid esters, esters of polyols having three or more hydroxy groups, esters of polycarboxylic acids having three or more carboxyl groups, polyol ethers, polyamines, and alcohols having six or more carbon atoms).

### Amount, etc. of ingredient (X)

The ingredient (X) falls under the category of type (1) and/or (2) as described above, and in the case of type (2), the ingredient (X) may satisfy at least one of the requirements (1) and (2) (in particular, at least the requirement (1)).

Specific examples of the ingredient (X) include the compounds exemplifying the ingredient (X) of type (1). Among these specific examples of the ingredient (X), for example, dicarboxylic acid esters (e.g., dicarboxylic acid diesters such as aliphatic dicarboxylic acid diesters), diol esters (e.g., diol diesters such as diesters of aliphatic diols having three or more carbon atoms), monocarboxylic acid esters (e.g., esters of aliphatic monocarboxylic acids such as esters of C₆₋₂₄ aliphatic carboxylic acids), esters of polyols having three or more hydroxy groups (e.g., triol triesters such as aliphatic triol tri-C₁₋₆ fatty acid esters), polyol ethers [e.g., diol diethers such as aliphatic diol diethers (such as dialkyl ethers)], polyamines [e.g., diamines such as N-alkyl substituted diamines (e.g., N,N'-dialkyldiamines)], alcohols having six or more carbon atoms [e.g., C₁₀₋₁₆ alkanols (e.g., straight-chained alkanols such as 1-decanol, 1-dodecanol, and 1-hexadecanol), aralkyl alcohols (e.g., benzyl alcohol), etc.], etc., are suitable from the perspective of the dissolution of the l-menthol and others.

Among these specific examples of the ingredient (X), dicarboxylic acid esters (e.g., aliphatic dicarboxylic acid diesters such as diesters of aliphatic dicarboxylic acids having four or more carbon atoms) are suitable from the perspective of compatibility (balance) between the dissolution of the l-menthol and the retention of l-menthol flavor.

Therefore, the ingredient (X) may at least comprise an ingredient (compound) described above.

Such an ingredient may or may not be an ingredient that falls under the category of type (2) (satisfies at least one of the requirements (1) and (2)), but is preferably an ingredient that falls under the category of type (2).

The amount (concentration) of the ingredient (X) in the composition can be selected according to the embodiment of the composition, the type of ingredient (X) selected, the application, and other factors, and is not particularly limited. The amount (concentration) of the ingredient (X) in the composition can usually be selected from a range of about 99 mass% or less (e.g., 97 mass% or less) and is, for example, 95 mass% (or % by weight or wt%) or less (e.g., 90 mass% or less), preferably 85 mass% or less (e.g., 80 mass% or less), more preferably 75 mass% or less (e.g., 70 mass% or less); or may be 65 mass% or less (e.g., 60 mass% or less, 55 mass% or less, 50 mass% or less, 45 mass% or less, 40 mass% or less, 35 mass% or less, 30 mass% or less, 25 mass% or less, 20 mass% or less, etc.) .

The lower limit of the amount (concentration) of the ingredient (X) can be selected according to the embodiment of the composition, the type of ingredient (X) (the ability to dissolve the l-menthol), the type and amounts of the additional ingredients (such as a carrier and a flavoring agent) to be combined, the application, and other factors, and is not particularly specified. For example, the lower limit may be 1 mass%, 2 mass%, 3 mass%, 4 mass%, 5 mass%, 6 mass%, 7 mass%, 8 mass%, 9 mass%, 10 mass%, 12 mass%, 15 mass%, 18 mass%, 20 mass%, 22 mass%, 25 mass%, etc.

The range of the amount (concentration) of the ingredient (X) may be set similarly as described above by combining these upper and lower limits as appropriate (e.g., 1 to 85 mass%, 5 to 50 mass%, 10 to 35 mass%, etc.).

The amount (concentration) of the ingredient (X) relative to the combined amount of the l-menthol and the ingredient (X) [when the combined amount is assumed as 100 mass%] can be selected from the above-described concentration ranges of the ingredient (X) in the composition (e.g., 90 mass% or less, 80 mass% or less, 75 mass% or less, 50 mass% or less, 45 mass% or less, 1 to 60 mass%, 2 to 35 mass%, 3 to 30 mass%, etc.).

The ingredient (X) is useful for dissolving the l-menthol, and when the ingredient (X) having a particularly excellent ability to dissolve the l-menthol is used, even a smaller amount of the ingredient (X) (e.g., 50 mass% or less, 30 mass% or less, 25 mass% or less, or 20 mass% or less when the combined amount is assumed as 100 mass%) enables dissolution of the l-menthol. The use of such a smaller amount of the ingredient (X) can lead to a higher concentration of l-menthol in the composition and thus is preferable.

### Additional ingredients

The composition of the present invention may comprise an additional ingredient in addition to the l-menthol and the ingredient (X).

The additional ingredient is not particularly limited and can be selected according to the form and application of the composition, the intended subject of the composition, and other factors. Examples include carriers, excipients, binders, disintegrants, lubricants, coating agents, colorants, flavoring agents, stabilizers, emulsifiers (surfactants), absorption enhancers, gelling agents, pH adjusters, preservatives, antioxidants, coolants, physiologically active substances, biologically active substances, microorganisms, foods and drinks, plants, sweeteners, acidulants, seasonings, and revitalizers.

A single kind of additional ingredient or a combination of two or more kinds of additional ingredients may be used.

Some types of ingredients (X) may function as such additional ingredients (e.g., as a flavoring agent).

Examples of the carrier (medium) include acids (e.g., fatty acids such as caprylic acid, capric acid, eicosapentaenoic acid, docosahexaenoic acid, oleic acid, and linoleic acid), fats or oils [e.g., vegetable oils (e.g., soybean oil, rapeseed oil, corn oil, sesame oil, linseed oil, cotton seed oil, perilla oil, olive oil, rice oil, palm oil, jojoba oil, sunflower oil, camellia oil, etc.), animal oils (e.g., beef fat, pork fat, chicken fat, milk fat, fish oil, horse oil, etc.), medium-chain triglycerides (MCTs), etc.], hydrocarbons (e.g., liquid paraffin, squalane, and vaseline), silicones (e.g., silicone oil, etc.), synthetic polymers (e.g., polyacrylic acid, carboxyvinyl polymer, polyethylene glycol, polyvinylpyrrolidone, etc.), natural polymers or their derivatives (e.g., carrageenan, alginic acid, cellulose, guar gum, xanthan gum, quince seed, dextran, gellan gum, hyaluronic acid, ethyl cellulose, hydroxypropyl cellulose, hydroxypropyl methyl cellulose, cationic guar gum, acetylated hyaluronic acid, sodium alginate, etc.), lower alcohols (e.g., ethanol, isopropanol, etc.), polyhydric alcohols (e.g., ethylene glycol, glycerol, propylene glycol, butylene glycol, diglycerol, and dipropylene glycol), and water.

The properties of the carrier can be selected according to the form of the composition and other factors. The carrier may be in a solid, liquid, or some other form, and may be non-volatile or volatile. A liquid carrier can be a solvent.

Among these carriers, fats or oils (particularly such as MCTs) are preferred. In addition, some types of fats or oils (e.g., MCTs) not only function as carriers, but also are useful for dissolution and flavor retention of the l-menthol in the composition and thus relatively easy to use. In addition, the use of fats or oils (such as MCTs) facilitates encapsulation.

The fatty acid (medium-chain fatty acid) constituting MCTs is not particularly limited, and examples include caproic acid, caprylic acid, capric acid, and lauric acid. The combination and ratio of the fatty acids (constituent fatty acids) of MCTs are also not particularly limited.

In the case where the carrier (or solvent, for example, a fat or oil such as an MCT) is used, the amount (concentration) of the carrier in the composition can be selected according to the embodiment of the composition, the type of ingredient (X) selected, the application, and other factors, and is not particularly limited. For example, the amount (concentration) of the carrier in the composition can be selected from a range of about 95 mass% or less (e.g., 90 mass% or less) and is, for example, 80 mass% or less, preferably 60 mass% or less, more preferably 50 mass% or less (e.g., 45 mass% or less); or may be 40 mass% or less (e.g., 35 mass% or less, 30 mass% or less, 25 mass% or less, 20 mass% or less, 15 mass% or less, 10 mass% or less, 8 mass% or less, 5 mass% or less, etc.).

The lower limit of the amount (concentration) of the carrier can be selected according to the embodiment of the composition, the type of ingredient (X) (the ability to dissolve the l-menthol), the application, and other factors, and is not particularly specified. For example, the lower limit may be 0.5 mass%, 1 mass%, 2 mass%, 3 mass%, 4 mass%, 5 mass%, 6 mass%, 7 mass%, 8 mass%, 9mass%, 10 mass%, 12 mass%, 15mass%, 18 mass%, 20 mass%, 22 mass%, 25 mass%, etc.

The range of the amount (concentration) of the carrier may be set similarly as described above by combining these upper and lower limits as appropriate (e.g., 1 to 50 mass%, 2 to 40 mass%, 3 to 25 mass%, etc.).

In the case where the carrier is used, the amount (concentration) of the carrier relative to the combined amount of the l-menthol, the ingredient (X), and the carrier [when the combined amount is assumed as 100 mass%] can be selected from the above-described concentration ranges of the carrier in the composition (e.g., 50 mass% or less, 40 mass% or less, 30 mass% or less, 1 to 50 mass%, 2 to 35 mass%, 3 to 30 mass%, etc.).

In the case where the carrier is used, the amount of the carrier can be selected according to the type of carrier, the type of ingredient (X) and its ability to dissolve the l-menthol, the concentration of the l-menthol, and other factors. For example, the amount of the carrier relative to 100 parts by mass of the ingredient (X) is, for example, about 1 to 10,000 parts by mass (e.g., 2 to 5,000 parts by mass), preferably about 5 to 2,000 parts by mass (e.g., 10 to 1,000 parts by mass), more preferably about 15 to 700 parts by mass (e.g., 20 to 500 parts by mass) ; or may be 200 parts by mass or less, 150 parts by mass or less, 100 parts by mass or less, 80 parts by mass or less, 50 parts by mass or less, 100 parts by mass or more, 120 parts by mass or more, 150 parts by mass or more, 200 parts by mass or more, etc.

The flavoring agent may be a synthetic or natural flavoring agent, a blended flavor, or a flavoring composition. The natural flavoring agent (the original source of the natural flavoring agent) can be, for example, from mint, herbs, citrus, and many others, and is not particularly limited.

Specific examples of the flavoring agent include hydrocarbons (e.g., ocimene, limonene, α-phellandrene, terpinene, 3-carene, bisabolene, valencene, alloocimene, myrcene, farnesene, α-pinene, β-pinene, camphene, terpinolene, p-cymene, cedrene, β-caryophyllene, and cadinene); and oils such as orange, neroli, mandarin, petitgrain, bergamot, tangerine, satsuma orange, cinnamon, daidai, hassak, iyokan, lemon, lime, grapefruit, yuzu, sudachi, kabosu, sweetie, raspberry, citronella, elemi, olibanum, marjoram, angelica root, star anise, basil, hay, calamus, caraway, cardamom, pepper, cascarilla, ginger, sage, clary sage, clove, coriander, eucalyptus, fennel, pimento, juniper, fenugreek, laurel, mace, Japanese cedar, senkyu, almond, anise, artemisia, alfalfa, apricot, ambrette, rush, strawberry, fig, ylang-ylang, wintergreen, ume, elder, enju, oakmoss, allspice, orris, currant, cassie, chamomile, galanga, Chinese quince, gambir, guava, gooseberry, camphor tree, gardenia, cubeb, cumin, cranberry, cola, Japanese pepper, sandarac, sandalwood, red sandalwood, perilla, civet, jasmine, ginseng, cinnamon, starfruit, styrax, spearmint, apple mint, peppermint, geranium, thyme, davana, tansy, champaca, tuberose, camellia, dittany, tolu balsam, tonka beans, nut, jujube, nutmeg, nanten, niaouli, carrot, violet, pineapple, hibiscus, honey, Japanese mint, passion fruit, vanilla, rose, coffee, hyssop, hinoki cypress, fusel oil, buchu, pepino, verbena, rosewood, pawpaw, boldo, boronia, pine, mango, beeswax, mimosa, milfoil (yarrow), musk, maple, melissa (lemonbalm), melon, peach, yara-yara, lavender, litsea, linden, rue, wax jambu, rosemary, and lovage.

In the case where the flavoring agent is used, the amount (concentration) of the flavoring agent in the composition can be selected according to the embodiment of the composition, the type of ingredient (X) selected, the application, and other factors, and is not particularly limited. For example, the amount (concentration) of the flavoring agent in the composition can be selected from a range of about 80 mass% or less (e.g., 60 mass% or less) and is, for example, 50 mass% or less (e.g., 40 mass% or less), preferably 30 mass% or less (e.g., 25 mass% or less), more preferably 20 mass% or less (e.g., 15 mass% or less), and particularly preferably 10 mass% or less (e.g., 8 mass% or less, 7 mass% or less, 6 mass% or less, 5 mass% or less, 4 mass% or less, 3 mass% or less, etc.).

The lower limit of the amount (concentration) of the flavoring agent can be selected according to the embodiment and application of the composition, and other factors, and is not particularly specified. For example, the lower limit may be 0.01 mass%, 0.1 mass%, 0.5mass%, 0.7 mass%, 1 mass%, 1.2 mass%, 1.5 mass%, 2 mass%, 3 mass%, etc.

The range of the amount (concentration) of the flavoring agent may be set similarly as described above by combining these upper and lower limits as appropriate (e.g., 0.1 to 30 mass%, 0.5 to 10 mass%, 1 to 5 mass%, etc.).

The flavoring agent (a flavoring agent other than l-menthol), when combined with the l-menthol in the composition, may disturb the flavor of (derived from) the l-menthol (generate or transform into an unusual odor). For this reason, it is desirable to use no flavoring agents (substantially no flavoring agents) when the composition is used for applications such as those in which l-menthol flavor is desired. If the flavoring agent is used, it is desirable that its amount is relatively small (e.g., 10 mass% or less in the composition, 5 mass% or less in the composition, etc.).

On the other hand, it is sometimes preferable from the perspective of flavor preference (e.g., flavor preference in smoking) and others to use a flavoring agent other than l-menthol, for example, for applications where l-menthol flavor is not desired and even for applications where l-menthol flavor is desired.

In such cases, the flavoring agent may be positively combined with the l-menthol, and the amount of the flavoring agent (a flavoring agent other than menthol) in the composition may be, for example, 1 mass% or more (e.g., 5 mass% or more, 10 mass% or more, 15 mass% or more, 20 mass% or more, etc.); and may be 90 mass% or less (e.g., 80 mass% or less, 70 mass% or less, 60 mass% or less, 50 mass% or less, 40 mass% or less, 30 mass% or less).

In the case where the flavoring agent is used, the amount of the flavoring agent relative to the combined amount of the l-menthol and the flavoring agent may be, for example, 1 mass% or more (e.g., 5 mass% or more, 10 mass%, 15 mass% or more, 20 mass% or more, 30 mass% or more, etc.); and may be 99 mass% or less (e.g., 95 mass% or less, 90 mass% or less, 85 mass% or less, 80 mass% or less, 70 mass% or less, 60 mass% or less, 50 mass% or less, or 40 mass% or less).

The emulsifier (surfactant) is not particularly limited, and examples include nonionic surfactants [e.g., sugar fatty acid esters (e.g., sucrose fatty acid esters, maltose fatty acid esters, and lactose fatty acid esters), propylene glycol fatty acid esters, glycerol fatty acid esters, sorbitan fatty acid esters, polyglycerol fatty acid esters, and organic acid monoglycerides].

In the case where the emulsifier is used, the amount (concentration) of the emulsifier in the composition can be selected according to the embodiment of the composition, the type of ingredient (X) selected, the application, and other factors, and is not particularly limited. For example, the amount (concentration) of the emulsifier in the composition can be selected from a range of about 40 mass% or less (e.g., 35 mass% or less) and is, for example, 30 mass% or less (e.g., 25 mass% or less), preferably 20 mass% or less (e.g., 15 mass% or less), and more preferably 10 mass% or less (e.g., 8 mass% or less) or 5 mass% or less (e.g., 4 mass% or less, 3 mass% or less, 1 mass% or less, etc.).

The lower limit of the amount (concentration) of the emulsifier can be selected according to the embodiment and application of the composition, and other factors, and is not particularly specified. For example, the lower limit may be 0.01 mass%, 0.1mass%, 0.5mass%, 0.7mass%, 1 mass%, 1.2 mass%, 1.5 mass%, 2 mass%, 3 mass%, etc.

The range of the amount (concentration) of the emulsifier may be set similarly as described above by combining these upper and lower limits as appropriate (e.g., 0.1 to 30 mass%, 0.5 to 10 mass%, 1 to 5 mass%, etc.).

The emulsifier can cause a significantly reduced interfacial tension in the composition. For this reason, when the composition comprising an emulsifier is used as a content of a capsule (e.g., a seamless capsule produced by a drop method or some other method), encapsulation is prone to failure. From this perspective, it is desirable to use no emulsifiers (substantially no emulsifiers) when the composition is used as a capsule content. If the emulsifier is used, it is desirable that its amount is relatively small (e.g., 5 mass% or less in the composition, 3 mass% or less in the composition, etc.).

### Composition

The composition may be formulated into an appropriate dosage form according to the desired application, the mode of delivery, and other factors. The form (dosage form, properties) of the composition (formulation) is not particularly limited, and examples include tablets, powders, fine granules, granules, dry syrups, coated tablets, orally disintegrating tablets, chewable tablets, capsules, soft capsules, syrups, oral liquids, lozenges, jellies, inhalations, suppositories, injections, ointments, eye drops, eye ointments, nasal drops, ear drops, patches, lotions, topical liquids, sprays, topical aerosols, creams, gels, tapes, buccal tablets, sublingual tablets, liquids, suspensions, emulsions, liniments, and sheets.

The composition may be a pharmaceutical product (medicament, pharmaceutical composition).

In the case of delivery of the composition (or l-menthol) into the body, the mode of delivery (administration or intake) is not particularly limited, and oral delivery (administration) or parenteral delivery (administration) may be selected. For parenteral delivery (administration), for example, pulmonary administration, nasal administration, dermal administration, mucosal (e.g., oral mucosal) administration, ocular instillation, auricular instillation, or injection (subcutaneous injection, intramuscular injection, intravenous injection, etc.) can be used. A single one of these modes of delivery or a combination of two or more of them may be used.

The composition of the present invention may be formulated into an appropriate dosage form as described above and can be used (applied) for various purposes (targets). Specific examples of the use (application) include capsules (e.g., capsule contents), filters, tobacco products, inhalation devices, perfumery or cosmetic products, food or drink products, and pharmaceutical products (medicaments, pharmaceutical compositions).

These examples will be described below.

### Capsules

The capsule may have a shell only or have a shell and a content (core). In particular, the capsule for tobacco products, etc., may have a core (content, inner liquid, inner substance) and a shell (outer layer, coat, capsule coat).

The capsule may be a soft capsule, a hard capsule, a seamless capsule, etc. In particular, the capsule for tobacco products, etc., may be a seamless capsule.

The composition (l-menthol and the ingredient (X)) may be contained in a capsule in any manner, whether in a shell, a core, or both. In particular, when the capsule is a capsule having a core (seamless capsule), the composition may be contained in the core (or at least in the core). In other words, the composition of the present invention is used for a capsule content.

The shell (outer layer) usually may contain a shell-forming component (shell-forming base, shell-forming material). The shell-forming component is not particularly limited and can be selected as appropriate according to the application of the capsule and other factors. Examples of the shell-forming component include polysaccharides (or their derivatives) {e.g., polysaccharides from seaweeds [e.g., agar, carrageenan, alginic acid or its salts (e.g., alkali metal salts (a sodium salt, a potassium salt, etc.), alkaline earth metal salts (a calcium salt, a magnesium salt, etc.), an iron salt, a tin salt, and other metal salts), furcellaran, curdlan, etc.], polysaccharides from resins (e.g., gum ghatti, gum arabic, etc.), polysaccharides from microorganisms (e.g., pullulan, welan gum, xanthan gum, gellan gum, etc.), polysaccharides from plants (e.g., tragacanth gum, pectin, glucomannan, starch, polydextrose, dextrin, maltodextrin, cyclodextrin, indigestible dextrin, etc.), polysaccharides from seeds [e.g., guar gum or its derivatives (e.g., hydroxypropyl guar gum, cationic guar gum, and hydrolyzed guar gum (such as enzymatically hydrolyzed guar gum)), tara gum, tamarind seed gum, locust bean gum, psyllium seed gum, and linseed gum], fermented polysaccharides (e.g., diutan gum, etc.), cellulose derivatives (e.g., hydroxypropyl cellulose, hydroxypropylmethyl cellulose, methyl cellulose, carboxymethyl cellulose, etc.), chitosan, etc.}, synthetic resins (polyvinyl alcohol, etc.), proteins (e.g., gelatin, casein, zein, etc.), and sugar alcohols (e.g., sorbitol, maltitol, lactitol, hydrogenated isomaltulose, xylitol, mannitol, galactitol, erythritol, etc.).

A single kind of shell-forming component or a combination of two or more kinds of shell-forming components may be used.

The shell-forming component may be capable of forming hydrophilic colloids. Some types of shell-forming components can function, for example, as a plasticizer, a sweetener, a dietary fiber, or a bulking agent. The shell-forming component may be a commercial product.

The shell may contain a plasticizer, a colorant, a sweetener, a flavoring agent, an antioxidant, a preservative, and other components.

For example, the shell may contain a plasticizer for purposes including adjustment of shell strength. Specific examples of the plasticizer include polyhydric alcohols (e.g., (poly)alkylene glycols such as ethylene glycol, propylene glycol, polyethylene glycol, and polypropylene glycol; and polyols having three hydroxy groups or more, such as glycerol), sugars [e.g., monosaccharides (e.g., glucose, fructose, glucose, galactose, etc.), disaccharides (e.g., sucrose, maltose, trehalose, coupling sugar, etc.), oligosaccharides (e.g., maltooligosaccharides, etc.), etc.], sugar alcohols (e.g., sugar alcohols as exemplified above, such as sorbitol, maltitol, lactitol, hydrogenated isomaltulose, xylitol, mannitol, galactitol, and erythritol), polysaccharides or their derivatives [e.g., starch, starch derivatives (e.g., polydextrose, dextrin, maltodextrin, indigestible dextrin, cyclodextrins (α, β, and γ), etc.), cellulose derivatives (e.g., hydroxypropyl cellulose, hydroxypropylmethyl cellulose, methyl cellulose, carboxymethyl cellulose, etc.), etc.], polyvinyl alcohol, and triacetin. A single kind of plasticizer or a combination of two or more kinds of plasticizers may be used.

Sugar alcohols, starch, starch derivatives, etc., can be used also as the shell-forming component as described above.

In the capsule having a core, the core may be in a solid, liquid, or some other form. Particularly in the capsule containing the composition of the present invention as a core, the core may be in a liquid form. The liquid form includes a colloidal form, an emulsified form, and a gelatinous form.

The core contains at least l-menthol and an ingredient (X) as described above, and may contain an additional ingredient.

The additional ingredient is, for example, a carrier [particularly, a liquid carrier (e.g., a liquid fat or oil such as an MCT)], a flavoring agent, or some other ingredient as exemplified above.

In such a capsule, the core is formed of the composition of the invention, and preferable embodiments, etc., of the core are as described above.

The core usually may be non-soluble in (non-erosive to) the shell (or to the area in contact with the shell).

The diameter (average diameter) of the capsule (or shell) can be selected as appropriate according to the type of capsule, the application, and other factors. The diameter may be, for example, 0.1 mm or more, 0.5 mm or more, 1 mm or more, 1.5 mm or more, 2 mm or more, etc. In addition, the diameter may be 30 mm or less, 25 mm or less, 20 mm or less, 18 mm or less, 15 mm or less, 12 mm or less, 10 mm or less, 8 mm or less, etc. More specifically, the diameter may be 2.8 mm, 3.0 mm, 3.4 mm, 3.5 mm, 4.0 mm, etc., but is not limited thereto.

The percentage of the shell in the capsule having a core (the proportion of the shell relative to the whole of the capsule (the combined amount of the shell and the capsule content)) may be selected from, for example, a range of about 0.1 to 99 mass% (e.g., 0.5 to 95 mass%). The shell percentage is, for example, about 1 to 90 mass%, preferably about 1.5 to 80 mass% (e.g., 2 to 70 mass%), and more preferably about 2.5 to 60 mass% (e.g., 3 to 50 mass%).

The thickness of the shell in the capsule having a core is not particularly limited and may be, for example, 1 to 200 µm, 3 to 150 µm, 5 to 100 µm, etc.

The capsule (e.g., the capsule having a core) may be breakable (disintegrable) (e.g., easily disintegrable or easily breakable). The crush strength of the capsule is determined according to the diameter and other factors, and may be, for example, 100 g or more, 200 g or more, 300 g or more, 400 g or more, 500 g or more, 600 g or more, 700 g or more, 800 g or more, 900 g or more, 1,000 g or more, etc.

The upper limit of the crush strength of the capsule is not particularly specified. The crush strength of the capsule may be, for example, 20,000 g or less, 15,000 g or less, 12,000 g or less, 10,000 g or less, etc.

The crush strength can be measured using, for example, a rheometer (CR-3000EX, manufactured by Sun Scientific).

The ratio of the crush strength (g) to the outer diameter (mm) (crush strength/outer diameter ratio) of the capsule (e.g., the capsule having a content) is not particularly limited and is, for example, 200 or more (e.g., more than 200), preferably 210 or more (e.g., 220 or more), more preferably 230 or more (e.g., 240 or more); or may be 250 or more, 300 or more, 400 or more, etc.

The upper limit of the ratio of the crush strength to the outer diameter (crush strength/outer diameter ratio) is not particularly specified and may be, for example, 20000, 15000, 10000, 8000, 6000, 5000, etc.

The ratio of the crush strength to the outer diameter can be said to be an index that reflects the practical breakability of the capsule, because there can be cases where the capsule is easily breakable even when the crush strength is high (for example, a case where the outer diameter is long).

The crush deformation of the capsule is determined according to the outer diameter and other factors, and may be, for example, 0.1 mm or more, 0.2 mm or more, 0.5 mm or more, 1.0 mm or more, etc.

The upper limit of the crush deformation of the soft capsule is not particularly specified. The crush deformation of the soft capsule may be, for example, 15 mm or less, 10 mm or less, 8 mm or less, etc.

The crush deformation can be measured using, for example, a rheometer (CR-3000EX, manufactured by Sun Scientific).

The ratio of the crush deformation (mm) to the outer diameter (mm) (crush deformation/outer diameter ratio) in the capsule is not particularly limited and is, for example, 0.1 or more, preferably 0.12 or more, more preferably 0.15 or more; or may be 0.18 or more, 0.2 or more, etc.

The upper limit of the ratio of the crush deformation to the outer diameter (crush deformation/outer diameter ratio) is not particularly specified and may be, for example, 1.0, 0.98, 0.97, 0.96, 0.95, etc.

The capsule may be used as it is or in combination with another capsule, depending on the application and other factors. The capsule may be, for example, embedded in a filter, as described later.

Said another capsule may be a capsule that does not contain l-menthol. Said another capsule is, for example, a capsule having a core and a shell in which neither the core nor the shell contains l-menthol (e.g., a capsule containing a flavoring agent other than l-menthol).

Capsules (e.g., seamless capsules) can be produced using known methods. The capsule production methods are described in, for example, Japanese Patent No. 5047285, JP-A H10-506841, Japanese Patent No. 5581446, etc. More specifically, a drop-in-liquid method using a double or multiple nozzle can be employed. In this method, capsule shells are filled with a capsule content liquid, and the shells are then cured and dried to produce seamless capsules.

### Filters

The mode of use of the composition in filters is not particularly limited. For example, the composition may be contained in (attached to) various parts of a filter (filtration material, filtration member).

In particular, this type of filter may be a capsule-containing filter (a capsule-embedded filter, or a filter composed of a capsule-embedded filtration member).

In other words, this filter contains a capsule containing the composition (l-menthol and the ingredient (X)), which capsule is referred to as a first capsule. The first capsule can be, for example, the same capsule as described in the section "Capsules" above. The first capsule is particularly preferably a capsule having a core and a shell, which core (capsule content) contains l-menthol and an ingredient (X) (is formed of the composition of the present invention).

The filter contains at least the first capsule, and may contain a second capsule, which is different from the first capsule.

The second capsule is not particularly limited as long as it is a capsule different from the first capsule. For example, the second capsule may be a capsule whose content is different from the content of the first capsule.

The second capsule is, for example, a capsule having a core and a shell, which core (and shell) contains a carrier and/or a flavoring agent (a flavoring agent other than l-menthol) (in particular, a capsule that does not contain l-menthol).

The capsule contained in the above-described type of filter can be the same capsule as described in the section "Capsules" above. The capsule that does not contain l-menthol (such as the second capsule) can be the same capsule as described in the section "Capsules" above except for the absence of l-menthol.

The filter is not particularly limited and may be, for example, a filter for air conditioners, air purifiers, etc.

In particular, the capsule-containing filter is suitable for use as a tobacco filter, etc. In a mode of use in tobacco filters, etc., l-menthol can efficiently be delivered via a pulmonary route.

### Tobacco products

The mode of use of the composition in tobacco products is not particularly limited. For example, the composition may be contained in (attached to) various parts of a tobacco product (tobacco leaf-containing part, tobacco filter, etc.).

Typically and preferably, a capsule or filter containing the composition is used in tobacco products.

As long as the capsule or filter is used in tobacco products, such tobacco products may be conventional tobacco products (combustible tobacco products) or non-combustible tobacco products [e.g., heated tobacco products (direct heated, air heated, etc.)].

### Inhalation devices

The mode of use of the composition in inhalation devices is not particularly limited. For example, the composition may be contained in (attached to) various parts of an inhalation device.

The inhalation device is not particularly limited, and examples include smoking devices and non-smoking devices.

Examples of the smoking device include heated tobacco products (such as vapor tobacco products), e-cigarettes, bongs (water pipes), and vaporizers. Heated tobacco products can be used for nicotine intake, while e-cigarettes are nicotine-free. The heated tobacco product is not particularly limited, and examples include IQOS (Philip Morris International), glo (British American Tobacco), Ploom S and Ploom TECH (Japan Tobacco), and Pulze (Imperial Tobacco Group plc). The e-cigarette is not particularly limited, and examples include ego AIO (Joytech) and ICE VAPE (Common Wealth).

The non-smoking device may be, for example, a medical device or a non-medical device (e.g., a health care device) . Specific examples of the non-smoking device include inhalers (e.g., nebulizers, steam inhalers), facial machines, and humidifiers.

More specifically, the composition (or the l-menthol and the ingredient (X)) may be contained in an inhalant in inhalation devices [e.g., a liquid solution in smoking devices such as heated tobacco products (such as vapor tobacco products) and e-cigarettes]. In a mode of use in inhalants, l-menthol can efficiently be delivered via a pulmonary route.

The inhalant (such as a liquid solution) may contain an additional ingredient in addition to the l-menthol and the ingredient (X). The inhalant usually may contain a carrier [a liquid carrier, for example, a polyhydric alcohol (e.g., glycerol, propylene glycol, etc.)], and if necessary, may further contain a flavoring agent (a flavoring agent other than l-menthol) (may be a flavored liquid solution).

The amounts of the l-menthol and other ingredients in the inhalant (such as a liquid solution) may be selected from the respective ranges as described above.

### Perfumery or cosmetic products

Perfumery or cosmetic products include air fresheners, oral care products (oral preparations, oral care preparations), cosmetic products, bath salts, perfumes, detergents, fabric softeners, toiletries, pesticides, and paints.

Examples of the air freshener include, but are not particularly limited to, liquid air fresheners and gel air fresheners.

Examples of the oral care product include dentifrices (e.g., toothpaste, gel toothpaste, liquid-state toothpaste, liquid toothpaste, moist toothpaste, etc.), mouthwashes, mouth fresheners, chewing gums, gummies, candies, chocolates, drinks, and tablet confectionaries.

Examples of the cosmetic product include, but are not particularly limited to, basic care products (e.g., lotions, milky lotions, gels, creams, serums, sunscreens, packs, masks, hand creams, body lotions, and body creams), cleansing products (e.g., facial washes, make-up removers, body shampoos, shampoos, conditioners, and treatments), make-up products (e.g., foundations, colors, lipsticks, and lip balms), and hair care products (e.g., tonics, creams, liquids, and sprays). The cosmetic product may be a skin care product.

The mode of use (blending or addition) of the composition in perfumery or cosmetic products is not particularly limited and can be selected according to the type of perfumery or cosmetic product and other factors. In a mode of use in perfumery or cosmetic products, l-menthol can efficiently be delivered via a pulmonary route.

The amounts of the l-menthol and other ingredients in the perfumery or cosmetic product may be selected from the respective ranges as described above.

### Food or drink products

Examples of the food or drink product include, but are not particularly limited to, capsules, drinks, foods (processed foods), and confectioneries.

The food or drink product may be, for example, a health food (e.g., a food for specified health uses or a food with nutrient function claims), a dietary supplement, a feed, or a food additive.

Examples of the capsule include, but are not particularly limited to, seamless capsules, soft capsules, and other capsules such as those in the examples above. The shell of the capsule and the embodiment of the capsule (such as a capsule shell) may be as exemplified above.

The mode of use of the composition (the l-menthol and the ingredient (X)) in food or drink products is not particularly limited and may be selected according to the embodiment of the food or drink product. For example, in the case of capsules as described above, the composition may be contained in the capsule (e.g., the capsule core and/or shell), or the composition may be added to (blended into) a food or drink product (the composition is used as an additive for a food or drink product).

In the case where the composition is added to a food or drink product, examples of the food or drink product include, but are not particularly limited to, foods [e.g., noodles (such as soba, udon, Chinese noodles, and instant noodles), confectioneries, breads, processed seafood or livestock products (such as fish cakes, hams, and sausages), dairy products (such as processed milk and fermented milk), fats or oils and processed fats or oils (such as salad oil, tempura oil, margarine, mayonnaise, shortening, whipped cream, and dressings), seasonings (such as sauces and tare sauces), retort pouch foods (such as curry, stew, rice bowl, porridge, and rice porridge), cold confectioneries (such as ice cream and sherbet), and fried foods]; and drinks (such as tea drinks, soft drinks, carbonated drinks, nutritional drinks, fruit drinks, and lactic acid drinks).

### Pharmaceutical products

Examples of the pharmaceutical product include, but are not particularly limited to, pharmaceutical products (compositions) containing menthol as an active ingredient. Specific examples of the pharmaceutical product include, for example, gastric peristalsis inhibitors (e.g., medicines containing menthol to be sprayed on the gastric wall during endoscopic examination).

### EXAMPLES

Hereinafter, the present invention will be described in detail by examples, but the present invention is not limited thereto.

The ingredients used in the examples were as follows.

The l-menthol used was a recrystallized product from steam-distilled essential oil from *Mentha canadensis*, which product was purchased from Anhui Tonghui Perfume Co., Ltd.

The peppermint oil used was a steam-distilled essential oil product from *Mentha piperita*, which product was purchased from Nagaoka & Co., Ltd.

The spearmint oil used was a steam-distilled essential oil product from *Mentha spicata*, which product was purchased from Nagaoka & Co., Ltd.

The MCT used was a pressed oil product from *Elaeis guineensis* fruits, which product was purchased from Kao Corporation.

Diethyl malonate, diethyl sebacate, diethyl fumarate, tributyrin, triethyl citrate, benzyl benzoate, ethyl decanoate, ethyl laurate, ethyl palmitate, ethylene glycol diacetate, 1,6-diacetoxyhexane, 1,8-diacetoxyhexane, diethylene glycol dibutyl ether, ethylene glycol dibutyl ether, N,N'-diethyl-1,6-hexanediamine, 1,2-diethoxyethane, 1-decanol, 1-dodecanol, 1-hexadecanol, d-limonene, and benzyl alcohol were purchased from FUJIFILM Wako Pure Chemical Corporation or Tokyo Chemical Industry Co., Ltd.

Diethyl succinate and diisobutyl adipate were purchased from Inoue Perfumery MFG. Co., Ltd.

In the examples, it will be understood that "parts by weight (pbw)" and "parts by mass (pbm)" have the same meaning.

### 1-Menthol dissolution test

For the l-menthol dissolution test, a total of 10 g of menthol and the other ingredients (solvent) was prepared and heated to 50°C to dissolve into a solution. The solution was stored at 20°C and evaluated based on the time during which the solution was maintained in a liquid state without solidifying.

A sample that solidified before being cooled down to 20°C [e.g., during the process of cooling down to a temperature below the melting point of l-menthol (generally 42 to 45°C)] or immediately (e.g., within 30 minutes) after being stored at 20°C was rated D. A sample that was maintained as a solution for a while (e.g., more than 30 minutes) but solidified in less than 24 hours was rated C. A sample that solidified in 24 hours or more but less than 10 days was rated B. A sample that did not solidify in 10 days or more was rated A.

The results are shown below.

### Experiment 1: 1-menthol + diethyl malonate

**[Table 4]**

| Dissolution of l-menthol in diethyl malonate used as solvent | | | | |
|---|---|---|---|---|
| l-Menthol (pbw) | 80 | 70 | 60 | 50 |
| Diethyl malonate (pbw) | 20 | 30 | 40 | 50 |
| Dissolution | B | B | B | B |

As shown in the table above, diethyl malonate used as a solvent efficiently dissolved menthol. More specifically, referring to the relationship with menthol concentration, diethyl malonate has an excellent ability (rated B from a scale of A to C, a moderate ability) to dissolve menthol regardless of the menthol concentration and is a preferable solvent.

### Experiment 2: 1-menthol + diethyl succinate

**[Table 5]**

| Dissolution of l-menthol in diethyl succinate used as a solvent | | | | |
|---|---|---|---|---|
| l-Menthol (pbw) | 80 | 70 | 60 | 50 |
| Diethyl succinate (pbw) | 20 | 30 | 40 | 50 |
| Dissolution | C | A | A | A |

As shown in the table above, diethyl succinate used as a solvent efficiently dissolved menthol. More specifically, referring to the relationship with menthol concentration, diethyl succinate has an excellent ability (rated A or B from a scale of A to C, a moderate to high ability) to dissolve menthol at a menthol concentration ranging 70 to 50 wt% and is a preferable solvent.

### Experiment 3: l-menthol + diisobutyl adipate

**[Table 6]**

| Dissolution of l-menthol in diisobutyl adipate used as a solvent | | | | |
|---|---|---|---|---|
| l-Menthol (pbw) | 80 | 70 | 60 | 50 |
| Diisobutyl adipate (pbw) | 20 | 30 | 40 | 50 |
| Dissolution | C | A | A | A |

As shown in the table above, diisobutyl adipate used as a solvent efficiently dissolved menthol. More specifically, referring to the relationship with menthol concentration, diisobutyl adipate has an excellent ability (rated A from a scale of A to C, a high ability) to dissolve menthol at a menthol concentration ranging 70 to 50 wt% and is a preferable solvent.

### Experiment 4: 1-menthol + diethyl sebacate

**[Table 7]**

| Dissolution of l-menthol in diethyl sebacate used as a solvent | | | | |
|---|---|---|---|---|
| l-Menthol (pbw) | 80 | 70 | 60 | 50 |
| Diethyl sebacate (pbw) | 20 | 30 | 40 | 50 |
| Dissolution | C | A | A | A |

As shown in the table above, diethyl sebacate used as a solvent efficiently dissolved menthol. More specifically, referring to the relationship with menthol concentration, diethyl sebacate has an excellent ability (rated A from a scale of A to C, a high ability) to dissolve menthol at a menthol concentration ranging 70 to 50 wt% and is a preferable solvent.

### Experiment 5: l-menthol + diethyl fumarate

**[Table 8]**

| Dissolution of l-menthol in diethyl fumarate used as a solvent | | | |
|---|---|---|---|
| l-Menthol (pbw) | 70 | 60 | 50 |
| Diethyl fumarate (pbw) | 30 | 40 | 50 |
| Dissolution | C | A | A |

As shown in the table above, diethyl fumarate used as a solvent efficiently dissolved menthol. More specifically, referring to the relationship with menthol concentration, diethyl fumarate has an excellent ability (rated A from a scale of A to C, a high ability) to dissolve menthol at a menthol concentration ranging 60 to 50 wt% and is a preferable solvent.

### Experiment 6: 1-menthol + tributyrin

**[Table 9]**

| Dissolution of l-menthol in tributyrin used as a solvent | | | |
|---|---|---|---|
| l-Menthol (pbw) | 70 | 60 | 50 |
| Tributyrin (pbw) | 30 | 40 | 50 |
| Dissolution | C | C | B |

As shown in the table above, tributyrin used as a solvent efficiently dissolved menthol. More specifically, referring to the relationship with menthol concentration, tributyrin has an excellent ability (rated B from a scale of A to C, a moderate ability) to dissolve menthol at a menthol concentration of 50% and is a preferable solvent.

### Experiment 7: l-menthol + triethyl citrate

**[Table 10]**

| Dissolution of l-menthol in triethyl citrate used as a solvent | | | |
|---|---|---|---|
| l-Menthol (pbw) | 70 | 60 | 50 |
| Triethyl citrate (pbw) | 30 | 40 | 50 |
| Dissolution | C | C | C |

As shown in the table above, triethyl citrate used as a solvent efficiently dissolved menthol.

### Experiment 8: l-menthol + benzyl benzoate

**[Table 11]**

| Dissolution of l-menthol in benzyl benzoate used as a solvent | | | |
|---|---|---|---|
| l-Menthol (pbw) | 70 | 60 | 50 |
| Benzyl benzoate (pbw) | 30 | 40 | 50 |
| Dissolution | C | C | B |

As shown in the table above, benzyl benzoate used as a solvent efficiently dissolved menthol. More specifically, referring to the relationship with menthol concentration, benzyl benzoate has an excellent ability (rated B from a scale of A to C, a moderate ability) to dissolve menthol at a menthol concentration of 50% and is a preferable solvent.

### Experiment 9: l-menthol + ethyl decanoate

**[Table 12]**

| Dissolution of l-menthol in ethyl decanoate used as a solvent | | | |
|---|---|---|---|
| l-Menthol (pbw) | 70 | 60 | 50 |
| Ethyl decanoate (pbw) | 30 | 40 | 50 |
| Dissolution | C | A | A |

As shown in the table above, ethyl decanoate used as a solvent efficiently dissolved menthol. More specifically, referring to the relationship with menthol concentration, ethyl decanoate has an excellent ability (rated A from a scale of A to C, a high ability) to dissolve menthol at a menthol concentration ranging 60 to 50 wt% and is a preferable solvent.

### Experiment 10: l-menthol + ethyl laurate

**[Table 13]**

| Dissolution of l-menthol in ethyl laurate used as a solvent | | | |
|---|---|---|---|
| l-Menthol (pbw) | 70 | 60 | 50 |
| Ethyl laurate (pbw) | 30 | 40 | 50 |
| Dissolution | C | A | A |

As shown in the table above, ethyl laurate used as a solvent efficiently dissolved menthol. More specifically, referring to the relationship with menthol concentration, ethyl laurate has an excellent ability (rated A from a scale of A to C, a high ability) to dissolve menthol at a menthol concentration ranging 60 to 50 wt% and is a preferable solvent.

### Experiment 11: 1-menthol + ethyl palmitate

**[Table 14]**

| Dissolution of l-menthol in ethyl palmitate used as a solvent | | | |
|---|---|---|---|
| l-Menthol (pbw) | 70 | 60 | 50 |
| Ethyl palmitate (pbw) | 30 | 40 | 50 |
| Dissolution | C | B | B |

As shown in the table above, ethyl palmitate used as a solvent efficiently dissolved menthol. More specifically, referring to the relationship with menthol concentration, ethyl palmitate has an excellent ability (rated B from a scale of A to C, a moderate ability) to dissolve menthol at a menthol concentration ranging 60 to 50% and is a preferable solvent.

### Experiment 12: l-menthol + ethylene glycol diacetate

**[Table 15]**

| Dissolution of l-menthol in ethylene glycol diacetate used as a solvent | | | |
|---|---|---|---|
| l-Menthol (pbw) | 70 | 60 | 50 |
| Ethylene glycol diacetate (pbw) | 30 | 40 | 50 |
| Dissolution | C | C | C |

As shown in the table above, ethylene glycol diacetate used as a solvent efficiently dissolved menthol.

### Experiment 13: l-menthol + 1,6-diacetoxyhexane

**[Table 16]**

| Dissolution of l-menthol in 1,6-diacetoxyhexane used as a solvent | | | |
|---|---|---|---|
| l-Menthol (pbw) | 70 | 60 | 50 |
| 1,6-Diacetoxyhexane (pbw) | 30 | 40 | 50 |
| Dissolution | C | A | A |

As shown in the table above, 1,6-diacetoxyhexane used as a solvent efficiently dissolved menthol. More specifically, referring to the relationship with menthol concentration, 1,6-diacetoxyhexane has an excellent ability (rated A from a scale of A to C, a high ability) to dissolve menthol at a menthol concentration ranging 60 to 50 wt% and is a preferable solvent.

### Experiment 14: l-menthol + 1,8-diacetoxyoctane

**[Table 17]**

| Dissolution of l-menthol in 1,8-diacetoxyoctane used as a solvent | | | |
|---|---|---|---|
| l-Menthol (pbw) | 70 | 60 | 50 |
| 1,8-Diacetoxyoctane (pbw) | 30 | 40 | 50 |
| Dissolution | C | C | A |

As shown in the table above, 1,8-diacetoxyoctane used as a solvent efficiently dissolved menthol. More specifically, referring to the relationship with menthol concentration, 1,8-diacetoxyoctane has an excellent ability (rated A from a scale of A to C, a high ability) to dissolve menthol at a menthol concentration of 50 wt% and is a preferable solvent.

### Experiment 15: l-menthol + diethylene glycol dibutyl ether

**[Table 18]**

| Dissolution of l-menthol in diethylene glycol dibutyl ether used as a solvent | | | |
|---|---|---|---|
| l-Menthol (pbw) | 70 | 60 | 50 |
| Diethylene glycol dibutyl ether (pbw) | 30 | 40 | 50 |
| Dissolution | C | A | A |

As shown in the table above, diethylene glycol dibutyl ether used as a solvent efficiently dissolved menthol. More specifically, referring to the relationship with menthol concentration, diethylene glycol dibutyl ether has an excellent ability (rated A from a scale of A to C, a high ability) to dissolve menthol at a menthol concentration ranging 60 to 50 wt% and is a preferable solvent.

### Experiment 16: l-menthol + ethylene glycol dibutyl ether

**[Table 19]**

| Dissolution of l-menthol in ethylene glycol dibutyl ether used as a solvent | | | |
|---|---|---|---|
| l-Menthol (pbw) | 70 | 60 | 50 |
| Ethylene glycol dibutyl ether (pbw) | 30 | 40 | 50 |
| Dissolution | A | A | A |

As shown in the table above, ethylene glycol dibutyl ether used as a solvent efficiently dissolved menthol. More specifically, referring to the relationship with menthol concentration, ethylene glycol dibutyl ether has an excellent ability (rated A from a scale of A to C, a high ability) to dissolve menthol at a menthol concentration ranging 70 to 50 wt% and is a preferable solvent.

### Experiment 17: l-menthol + N,N'-diethyl-1,6-exanediamine

**[Table 20]**

| Dissolution of l-menthol in N,N'-diethyl-1,6-hexanediamine used as a solvent | | | |
|---|---|---|---|
| l-Menthol (pbw) | 70 | 60 | 50 |
| N,N'-Diethyl-1,6-hexanediamine (pbw) | 30 | 40 | 50 |
| Dissolution | A | A | A |

As shown in the table above, N,N'-diethyl-1,6-exanediamine used as a solvent efficiently dissolved menthol. More specifically, referring to the relationship with menthol concentration, N,N'-diethyl-1,6-exanediamine has an excellent ability (rated A from a scale of A to C, a high ability) to dissolve menthol at a menthol concentration ranging 70 to 50 wt% and is a preferable solvent.

### Experiment 18: l-menthol + 1,2-diethoxyethane

**[Table 21]**

| Dissolution of l-menthol in 1,2-diethoxyethane used as a solvent | | | |
|---|---|---|---|
| l-Menthol (pbw) | 70 | 60 | 50 |
| 1,2-Diethoxyethane (pbw) | 30 | 40 | 50 |
| Dissolution | A | A | A |

As shown in the table above, 1,2-diethoxyethane used as a solvent efficiently dissolved menthol. More specifically, referring to the relationship with menthol concentration, 1,2-diethoxyethane has an excellent ability (rated A from a scale of A to C, a high ability) to dissolve menthol at a menthol concentration ranging 70 to 50 wt% and is a preferable solvent.

### Experiment 19: l-menthol + diethyl succinate + MCT

**[Table 22]**

| Dissolution of l-menthol in a mixture of diethyl succinate and MCT used as a solvent | | | | | | | | | |
|---|---|---|---|---|---|---|---|---|---|
| l-Menthol (pbw) | 70 | 70 | 70 | 65 | 65 | 65 | 60 | 60 | 60 |
| Diethyl succinate (pbw) | 10 | 15 | 20 | 10 | 15 | 20 | 10 | 15 | 20 |
| MCT (pbw) | 20 | 15 | 10 | 25 | 20 | 15 | 30 | 25 | 20 |
| Dissolution | C | C | B | C | C | A | A | A | A |

As shown in the table above, diethyl succinate efficiently dissolved menthol, when used in combination with or in place of the MCT. More specifically, referring to the relationship with menthol concentration, a mixed solvent of diethyl succinate and the MCT has a high ability (rated A from a scale of A to C) to dissolve menthol, for example, when the menthol concentration is 70 wt%, the diethyl succinate concentration is 20 wt%, and the MCT concentration is 10 wt%, when the menthol concentration is 65 wt%, the diethyl succinate concentration is 20 wt%, and the MCT concentration is 15 wt%, and when the menthol concentration is 60 wt%.

### Experiment 20: l-menthol + diethyl succinate + 1-decanol + MCT

**[Table 23]**

| Dissolution of l-menthol in a mixture of diethyl succinate, 1-decanol, and MCT used as a solvent | | | | |
|---|---|---|---|---|
| l-Menthol (pbw) | 75 | 75 | 70 | 65 |
| Diethyl succinate (pbw) | 10 | 15 | 15 | 15 |
| 1-Decanol (pbw) | 15 | 10 | 10 | 2 |
| MCT (pbw) | 0 | 0 | 5 | 23 |
| Dissolution | A | A | A | A |

As shown in the table above, diethyl succinate and 1-decanol efficiently dissolved menthol, when used in combination with or in place of the MCT. More specifically, referring to the relationship with menthol concentration, a mixed solvent of diethyl succinate, 1-decanol, and the MCT has a high ability (rated A from a scale of A to C) to dissolve menthol, for example, at a menthol concentration ranging 75 to 65 wt%.

### Experiment 21: l-menthol + diethyl succinate + 1-dodecanol + MCT

**[Table 24]**

| Dissolution of I-menthol in a mixture of diethyl succinate, 1-dodecanol, and MCT used as a solvent | | | | | | |
|---|---|---|---|---|---|---|
| I-Menthol (pbw) | 80 | 80 | 75 | 75 | 70 | 65 |
| Diethyl succinate (pbw) | 10 | 15 | 10 | 15 | 15 | 15 |
| 1-Dodecanol (pbw) | 10 | 5 | 15 | 10 | 10 | 10 |
| MCT (pbw) | 0 | 0 | 0 | 0 | 5 | 10 |
| Dissolution | A | A | A | A | A | A |

As shown in the table above, diethyl succinate and 1-dodecanol efficiently dissolved menthol, when used in combination with or in place of the MCT. More specifically, referring to the relationship with menthol concentration, a mixed solvent of diethyl succinate, 1-dodecanol, and the MCT has a high ability (rated A from a scale of A to C) to dissolve menthol, for example, at a menthol concentration ranging 80 to 65 wt%.

### Experiment 22: l-menthol + diethyl succinate/diisobutyl adipate/diethyl sebacate + 1-dodecanol

**[Table 25]**

| Dissolution of I-menthol in a mixture of 1-dodecanol with diethyl succinate, diisobutyl adipate, or diethyl sebacate used as a solvent | | | | | | | | | |
|---|---|---|---|---|---|---|---|---|---|
| I-Menthol (pbw) | 70 | 65 | 60 | 70 | 65 | 60 | 70 | 65 | 60 |
| Diethyl succinate (pbw) | 26 | 31 | 36 | 0 | 0 | 0 | 0 | 0 | 0 |
| Diisobutyl adipate (pbw) | 0 | 0 | 0 | 26 | 31 | 36 | 0 | 0 | 0 |
| Diethyl sebacate (pbw) | 0 | 0 | 0 | 0 | 0 | 0 | 26 | 31 | 36 |
| 1-Dodecanol (pbw) | 4 | 4 | 4 | 4 | 4 | 4 | 4 | 4 | 4 |
| Dissolution | A | A | A | A | A | A | B | B | B |

As shown in the table above, 1-dodecanol efficiently dissolved menthol, when used in combination with or in place of the other ingredient. More specifically, referring to the relationship with menthol concentration, a mixed solvent of 1-dodecanol with diethyl succinate or diisobutyl adipate has a high ability (rated A from a scale of A to C) to dissolve menthol, for example, at a menthol concentration ranging 70 to 60 wt%. In addition, a mixed solvent of 1-dodecanol with diethyl sebacate has a moderate ability (rated B from a scale of A to C) to dissolve menthol at a menthol concentration ranging 70 to 60 wt%.

### Experiment 23: 1-menthol + diethyl succinate + 1-hexadecanol + MCT

**[Table 26]**

| Dissolution of I-menthol in a mixture of diethyl succinate, 1-hexadecanol, and MCT used as a solvent | | | | |
|---|---|---|---|---|
| I-Menthol (pbw) | 75 | 75 | 70 | 65 |
| Diethyl succinate (pbw) | 10 | 15 | 15 | 15 |
| 1-Hexadecanol (pbw) | 15 | 10 | 10 | 2 |
| MCT (pbw) | 0 | 0 | 5 | 23 |
| Dissolution | A | B | A | A |

As shown in the table above, 1-hexadecanol efficiently dissolved menthol, when used in combination with or in place of the other ingredients. More specifically, referring to the relationship with menthol concentration, a mixed solvent of diethyl succinate, 1-hexadecanol, and the MCT has a moderate to high ability (rated A or B from a scale of A to C) to dissolve menthol, for example, at a menthol concentration ranging 75 to 65 wt%.

### Experiment 24: l-menthol + diethyl succinate + spearmint oil

**[Table 27]**

| Dissolution of I-menthol in a mixture of diethyl succinate and spearmint oil used as a solvent | | | | | | |
|---|---|---|---|---|---|---|
| I-Menthol (pbw) | 80 | 80 | 70 | 70 | 60 | 60 |
| Diethyl succinate (pbw) | 17 | 15 | 27 | 25 | 37 | 35 |
| Spearmint oil (pbw) | 3 | 5 | 3 | 5 | 3 | 5 |
| Dissolution | A | A | A | A | A | A |

As shown in the table above, diethyl succinate efficiently dissolved menthol, when used in combination with or in place of the other ingredient. More specifically, referring to the relationship with menthol concentration, a mixed solvent of diethyl succinate and spearmint oil has a high ability (rated A from a scale of A to C) to dissolve menthol, for example, at a menthol concentration ranging 80 to 60 wt%.

### Experiment 25: l-menthol + MCT

**[Table 28]**

| Dissolution of I-menthol in MCT used as a solvent | | | | | | | | |
|---|---|---|---|---|---|---|---|---|
| I-Menthol (pbw) | 70 | 65 | 60 | 55 | 50 | 45 | 40 | 35 |
| MCT (pbw) | 30 | 35 | 40 | 45 | 50 | 55 | 60 | 65 |
| Dissolution | C | C | C | B | B | B | A | A |

As shown in the table above, the MCT efficiently dissolved menthol as previously known. More specifically, referring to the relationship with menthol concentration, the MCT used as a solvent has a stably high ability to dissolve menthol, for example, at a menthol concentration of 40 wt% or less.

### Experiment 26: l-menthol + benzyl alcohol + MCT

**[Table 29]**

| Dissolution of I-menthol in a mixture of benzyl alcohol and MCT used as a solvent | | | | | |
|---|---|---|---|---|---|
| I-Menthol (pbw) | 80 | 70 | 70 | 65 | 60 |
| Benzyl alcohol (pbw) | 20 | 5 | 10 | 5 | 5 |
| MCT (pbw) | 0 | 25 | 20 | 30 | 35 |
| Dissolution | A | A | A | A | A |

As shown in the table above, benzyl alcohol efficiently dissolved menthol, when used alone or in combination with or in place of the MCT. More specifically, referring to the relationship with menthol concentration, benzyl alcohol alone or a mixed solvent of benzyl alcohol and the MCT has a high ability (rated A from a scale of A to C) to dissolve menthol, for example, at a menthol concentration ranging 80 to 60 wt%.

### Odor comparison test A

For the odor comparison test A, a total of 10 g of menthol and the other ingredients (solvent) were prepared as a sample, and the odor of the sample was compared with that of an MCT solution of menthol (a mixture of 45 wt% menthol and 55 wt% MCT) in sensory testing. Sensory testing was performed by a single person, who rated samples based on the following criteria: when a sample smells the same as the MCT solution of menthol, the sample is regarded as having no unusual odor; when a sample smells weakly of what is different from menthol, the sample is regarded as having a weak unusual odor; and when a sample smells strongly of what is distinctively different from menthol, the sample is regarded as having a strong unusual odor.

The results are shown below.

### Experiment A1: 1-menthol + diethyl malonate

**[Table 30]**

| | | | | |
|---|---|---|---|---|
| I-Menthol (pbw) | 80 | 70 | 60 | 50 |
| Diethyl malonate (pbw) | 20 | 30 | 40 | 50 |
| Odor (unusual odor) | None | None | Weak | Strong |

A sample containing 40 wt% diethyl malonate had a weak unusual odor, and a sample containing 50 wt% diethyl malonate had a distinct unusual odor, which was perceived as different from menthol flavor.

### Experiment A2: 1-menthol + diethyl succinate

**[Table 31]**

| | | | | |
|---|---|---|---|---|
| I-Menthol (pbw) | 80 | 70 | 60 | 50 |
| Diethyl succinate (pbw) | 20 | 30 | 40 | 50 |
| Odor (unusual odor) | None | None | None | None |

Nothing but menthol flavor was perceived at any ratio of the ingredients.

### Experiment A3: 1-menthol + diisobutyl adipate

**[Table 32]**

| | | | | |
|---|---|---|---|---|
| l-Menthol (pbw) | 80 | 70 | 60 | 50 |
| Diisobutyl adipate (pbw) | 20 | 30 | 40 | 50 |
| Odor (unusual odor) | None | None | None | None |

Nothing but menthol flavor was perceived at any ratio of the ingredients.

### Experiment A4: 1-menthol + diethyl sebacate

**[Table 33]**

| | | | | |
|---|---|---|---|---|
| I-Menthol (pbw) | 80 | 70 | 60 | 50 |
| Diethyl sebacate (pbw) | 20 | 30 | 40 | 50 |
| Odor (unusual odor) | None | None | None | None |

Nothing but menthol flavor was perceived at any ratio of the ingredients.

### Experiment A5: 1-menthol + diethyl succinate + MCT

**[Table 34]**

| | | | | | | | | | | | | |
|---|---|---|---|---|---|---|---|---|---|---|---|---|
| I-Menthol (pbw) | 70 | 70 | 70 | 70 | 65 | 65 | 65 | 65 | 60 | 60 | 60 | 60 |
| Diethyl succinate (pbw) | 10 | 15 | 20 | 25 | 10 | 15 | 20 | 25 | 10 | 15 | 20 | 25 |
| MCT (pbw) | 20 | 15 | 10 | 5 | 25 | 20 | 15 | 10 | 30 | 25 | 20 | 15 |
| Odor (unusual odor) | None | None | None | None | None | None | None | None | None | None | None | None |

Nothing but menthol flavor was perceived at any ratio of the ingredients.

### Experiment A6: 1-menthol + diethyl succinate + 1-dodecanol + MCT

**[Table 35]**

| | | | | | | |
|---|---|---|---|---|---|---|
| l-Menthol (pbw) | 80 | 80 | 75 | 75 | 70 | 65 |
| Diethyl succinate (pbw) | 10 | 15 | 10 | 15 | 15 | 15 |
| 1-Dodecanol (pbw) | 10 | 5 | 15 | 10 | 10 | 10 |
| MCT (pbw) | 0 | 0 | 0 | 0 | 5 | 10 |
| Odor (unusual odor) | None | None | None | None | None | None |

Nothing but menthol flavor was perceived at any ratio of the ingredients.

### Experiment A7: 1-menthol + diethyl succinate/diisobutyl adipate/diethyl sebacate + 1-dodecanol

**[Table 36]**

| | | | | | | | | | |
|---|---|---|---|---|---|---|---|---|---|
| I-Menthol (pbw) | 70 | 65 | 60 | 70 | 65 | 60 | 70 | 65 | 60 |
| Diethyl succinate (pbw) | 26 | 31 | 36 | 0 | 0 | 0 | 0 | 0 | 0 |
| Diisobutyl adipate (pbw) | 0 | 0 | 0 | 26 | 31 | 36 | 0 | 0 | 0 |
| Diethyl sebacate (pbw) | 0 | 0 | 0 | 0 | 0 | 0 | 26 | 31 | 36 |
| 1-Dodecanol (pbw) | 4 | 4 | 4 | 4 | 4 | 4 | 4 | 4 | 4 |
| Odor (unusual odor) | None | None | None | None | None | None | None | None | None |

Nothing but menthol flavor was perceived at any ratio of the ingredients.

### Experiment A8: 1-menthol + diethyl succinate + 1-hexadecanol + MCT

**[Table 37]**

| | | | | |
|---|---|---|---|---|
| I-Menthol (pbw) | 75 | 75 | 70 | 65 |
| Diethyl succinate (pbw) | 10 | 15 | 15 | 15 |
| 1-Hexadecanol (pbw) | 15 | 10 | 10 | 2 |
| MCT (pbw) | 0 | 0 | 5 | 23 |
| Odor (unusual odor) | None | None | None | None |

Nothing but menthol flavor was perceived at any ratio of the ingredients.

### Experiment A9

**[Table 38]**

| l-Menthol + diethyl succinate + spearmint oil | | | | | | |
|---|---|---|---|---|---|---|
| l-Menthol (pbw) | 80 | 80 | 70 | 70 | 60 | 60 |
| Diethyl succinate (pbw) | 17 | 15 | 27 | 25 | 37 | 35 |
| Spearmint oil (pbw) | 3 | 5 | 3 | 5 | 3 | 5 |
| Odor (unusual odor) | Weak | Strong | Weak | Strong | Weak | Strong |

Samples containing 3 wt% or more spearmint oil had an unusual odor, which was perceived as different from menthol flavor. At the same time, a flavor change from the simple menthol flavor was sensed.

### Experiment A10: 1-menthol + MCT

**[Table 39]**

| | | | | | | | | |
|---|---|---|---|---|---|---|---|---|
| I-Menthol (pbw) | 70 | 65 | 60 | 55 | 50 | 45 | 40 | 35 |
| MCT (pbw) | 30 | 35 | 40 | 45 | 50 | 55 | 60 | 65 |
| Odor (unusual odor) | None | None | None | None | None | None | None | None |

Nothing but menthol flavor was perceived at any ratio of the ingredients.

### Experiment A11: 1-menthol + limonene + MCT

**[Table 40]**

| | | | | | |
|---|---|---|---|---|---|
| I-Menthol (pbw) | 80 | 70 | 70 | 65 | 60 |
| d-Limonene (pbw) | 20 | 5 | 10 | 5 | 5 |
| MCT (pbw) | 0 | 25 | 20 | 30 | 35 |
| Odor (unusual odor) | Strong | Strong | Strong | Strong | Strong |

An unusual odor, which was different from menthol flavor, was perceived at all ratios of the ingredients.

### Experiment A12: 1-menthol + benzyl alcohol + MCT

**[Table 41]**

| | | | | | |
|---|---|---|---|---|---|
| I-Menthol (pbw) | 80 | 70 | 70 | 65 | 60 |
| Benzyl alcohol (pbw) | 20 | 5 | 10 | 5 | 5 |
| MCT (pbw) | 0 | 25 | 20 | 30 | 35 |
| Odor (unusual odor) | Strong | Weak | Weak | Weak | Weak |

An unusual odor, which was different from menthol flavor, was perceived at all ratios of the ingredients.

### Odor comparison test B

For the odor comparison test B, a total of 10 g of menthol and the other ingredients (solvent) were prepared as a sample, and the odor of the sample was compared with that of menthol in sensory testing.

Sensory testing was performed by eight well-trained persons, who rated samples based on the presence or absence of unusual odor on a scale up to 5 points. That is, when a sample smells the same as menthol (a mixture of 45 wt% menthol and 55 wt% MCT), the sample is given 5 points; when a sample smells weakly of what is different from menthol, the sample is given 3 points; when a sample smells strongly of what is distinctively different from menthol, the sample is given 1 point; when the average score is 4 points or more, the sample is rated A; when the average score is 3.5 points or more but less than 4 points, the sample is rated B; when the average score is 3 points or more but less than 3.5 points, the sample is rated C; when the average score is 2 points or more but less than 3 points, the sample is rated D; and when the average score is less than 2 points, the sample is rated E.

The results are shown below.

### Experiment B1: 1-menthol + diethyl malonate/diethyl succinate/diisobutyl adipate/diethyl sebacate

**[Table 42]**

| | | | | | | | |
|---|---|---|---|---|---|---|---|
| I-Menthol (pbw) | 70 | 60 | 50 | 50 | 50 | 60 | 70 |
| Diethyl malonate (pbw) | 30 | 40 | 50 | 0 | 0 | 0 | 0 |
| Diethyl succinate (pbw) | 0 | 0 | 0 | 50 | 0 | 0 | 0 |
| Diisobutyl adipate (pbw) | 0 | 0 | 0 | 0 | 50 | 0 | 0 |
| Diethyl sebacate (pbw) | 0 | 0 | 0 | 0 | 0 | 40 | 30 |
| Sensory test score | 3.00 | 3.00 | 2.25 | 4.00 | 3.50 | 4.25 | 4.13 |
| Rating | A | A | B | A | B | A | A |

### Experiment B2: 1-menthol + diethyl fumarate

**[Table 43]**

| | | | |
|---|---|---|---|
| I-Menthol (pbw) | 70 | 60 | 50 |
| Diethyl fumarate (pbw) | 30 | 40 | 50 |
| Sensory test score | 2.00 | 1.75 | 1.00 |
| Rating | D | E | E |

### Experiment B3: 1-menthol + tributyrin

**[Table 44]**

| | | |
|---|---|---|
| I-Menthol (pbw) | 60 | 50 |
| Tributyrin (pbw) | 40 | 50 |
| Sensory test score | 3.88 | 3.50 |
| Rating | B | B |

### Experiment B4: 1-menthol + triethyl citrate

**[Table 45]**

| | | | |
|---|---|---|---|
| l-Menthol (pbw) | 70 | 60 | 50 |
| Triethyl citrate (pbw) | 30 | 40 | 50 |
| Sensory test score | 4.00 | 3.75 | 3.63 |
| Rating | A | B | B |

### Experiment B5: 1-menthol + benzyl benzoate

**[Table 46]**

| | | | |
|---|---|---|---|
| I-Menthol (pbw) | 70 | 60 | 50 |
| Benzyl benzoate (pbw) | 30 | 40 | 50 |
| Sensory test score | 4.00 | 3.75 | 3.88 |
| Rating | A | B | B |

### Experiment B6: 1-menthol + ethyl laurate/ethyl palmitate

**[Table 47]**

| | | | | | |
|---|---|---|---|---|---|
| I-Menthol (pbw) | 70 | 60 | 50 | 70 | 50 |
| Ethyl laurate (pbw) | 30 | 40 | 50 | 0 | 0 |
| Ethyl palmitate (pbw) | 0 | 0 | 0 | 30 | 50 |
| Sensory test score | 3.75 | 3.00 | 2.25 | 3.38 | 3.25 |
| Rating | B | C | D | B | C |

### Experiment B7: 1-menthol + ethylene glycol diacetate/1,6-diacetoxyhexane/1,8-diacetoxyoctane

**[Table 48]**

| | | | | | | |
|---|---|---|---|---|---|---|
| I-Menthol (pbw) | 70 | 60 | 50 | 60 | 50 | 70 |
| Ethylene glycol diacetate (pbw) | 30 | 40 | 50 | 0 | 0 | 0 |
| 1,6-Diacetoxyhexane (pbw) | 0 | 0 | 0 | 40 | 50 | 0 |
| 1,8-Diacetoxyoctane (pbw) | 0 | 0 | 0 | 0 | 0 | 30 |
| Sensory test score | 2.63 | 2.50 | 2.75 | 3.00 | 2.63 | 2.00 |
| Rating | D | D | D | C | D | D |

### Experiment B8: 1-menthol + diethylene glycol dibutyl ether

**[Table 49]**

| | | | |
|---|---|---|---|
| I-Menthol (pbw) | 70 | 60 | 50 |
| Diethylene glycol dibutyl ether (pbw) | 30 | 40 | 50 |
| Sensory test score | 2.25 | 2.50 | 2.25 |
| Rating | D | D | D |

### Experiment B9: 1-menthol + N,N'-diethyl-1,6-hexanediamine

**[Table 50]**

| | | |
|---|---|---|
| I-Menthol (pbw) | 70 | 50 |
| N,N'-Diethyl-1,6-hexanediamine (pbw) | 30 | 50 |
| Sensory test score | 1.63 | 1.25 |
| Rating | E | E |

### Experiment B10: 1-menthol + diethyl succinate/diisobutyl adipate + dodecanol

**[Table 51]**

| | | | | |
|---|---|---|---|---|
| I-Menthol (pbw) | 80 | 80 | 80 | 80 |
| Diethyl succinate (pbw) | 10 | 15 | 0 | 0 |
| Diisobutyl adipate (pbw) | 0 | 0 | 10 | 15 |
| Dodecanol (pbw) | 10 | 5 | 10 | 5 |
| Sensory test score | 4.00 | 4.25 | 3.88 | 4.38 |
| Rating | A | A | B | A |

### Experiment B11: 1-menthol + diethyl succinate + dodecanol/hexadecanol

**[Table 52]**

| | | | | | |
|---|---|---|---|---|---|
| I-Menthol (pbw) | 70 | 60 | 70 | 65 | 60 |
| Diethyl succinate (pbw) | 26 | 36 | 26 | 31 | 36 |
| 1-Dodecanol (pbw) | 4 | 4 | 0 | 0 | 0 |
| 1-Hexadecanol (pbw) | 0 | 0 | 4 | 4 | 4 |
| Sensory test score | 3.25 | 3.38 | 4.13 | 4.50 | 4.50 |
| Rating | C | C | A | A | A |

### Experiment B12: 1-menthol + diisobutyl adipate + decanol/dodecanol/hexadecanol

**[Table 53]**

| | | | | | | | |
|---|---|---|---|---|---|---|---|
| I-Menthol (pbw) | 65 | 70 | 65 | 60 | 70 | 65 | 60 |
| Diisobutyl adipate (pbw) | 31 | 26 | 31 | 36 | 26 | 31 | 36 |
| 1-Decanol (pbw) | 4 | 0 | 0 | 0 | 0 | 0 | 0 |
| 1-Dodecanol (pbw) | 0 | 4 | 4 | 4 | 0 | 0 | 0 |
| 1-Hexadecanol (pbw) | 0 | 0 | 0 | 0 | 4 | 4 | 4 |
| Sensory test score | 3.25 | 4.13 | 4.50 | 4.13 | 4.50 | 4.63 | 4.63 |
| Rating | C | A | A | A | A | A | A |

### Experiment B13: 1-menthol + diethyl sebacate + decanol/dodecanol/hexadecanol

**[Table 54]**

| | | | | | | | |
|---|---|---|---|---|---|---|---|
| I-Menthol (pbw) | 65 | 70 | 65 | 60 | 70 | 65 | 60 |
| Diethyl sebacate (pbw) | 31 | 26 | 31 | 36 | 26 | 31 | 36 |
| 1-Decanol (pbw) | 4 | 0 | 0 | 0 | 0 | 0 | 0 |
| 1-Dodecanol (pbw) | 0 | 4 | 4 | 4 | 0 | 0 | 0 |
| 1-Hexadecanol (pbw) | 0 | 0 | 0 | 0 | 4 | 4 | 4 |
| Sensory test score | 3.13 | 4.13 | 4.13 | 4.25 | 4.13 | 4.25 | 4.38 |
| Rating | C | A | A | A | A | A | A |

### Experiment B14: 1-menthol + diethyl succinate/diisobutyl adipate + spearmint oil

**[Table 55]**

| | | | | |
|---|---|---|---|---|
| I-Menthol (pbw) | 80 | 80 | 70 | 60 |
| Diethyl succinate (pbw) | 16 | 0 | 0 | 0 |
| Diisobutyl adipate (pbw) | 0 | 16 | 26 | 36 |
| Spearmint oil (pbw) | 4 | 4 | 4 | 4 |
| Sensory test score | 3.50 | 4.00 | 4.88 | 4.00 |
| Rating | B | A | A | A |

### Experiment B15: 1-menthol + MCT

**[Table 56]**

| | | | |
|---|---|---|---|
| I-Menthol (pbw) | 60 | 50 | 40 |
| MCT (pbw) | 40 | 50 | 60 |
| Sensory test score | 4.13 | 4.75 | 4.63 |
| Rating | A | A | A |

### Experiment B16: 1-menthol + limonene + MCT

**[Table 57]**

| | | | | | |
|---|---|---|---|---|---|
| I-Menthol (pbw) | 80 | 70 | 70 | 65 | 60 |
| d-Limonene (pbw) | 20 | 5 | 10 | 5 | 5 |
| MCT (pbw) | 0 | 25 | 20 | 30 | 35 |
| Sensory test score | 2.00 | 2.25 | 2.38 | 2.25 | 2.63 |
| Rating | D | D | D | D | D |

### Experiment B17: 1-menthol + benzyl alcohol + MCT

**[Table 58]**

| | | | | | |
|---|---|---|---|---|---|
| I-Menthol (pbw) | 80 | 70 | 70 | 65 | 60 |
| Benzyl alcohol (pbw) | 20 | 5 | 10 | 5 | 5 |
| MCT (pbw) | 0 | 25 | 20 | 30 | 35 |
| Sensory test score | 1.75 | 2.38 | 2.13 | 2.38 | 2.13 |
| Rating | E | D | D | D | D |

### Encapsulation test (Encapsulation capability test) C

For the encapsulation test C, a total of 80 g of menthol and the other ingredients (solvent) were prepared as a sample, and the interfacial tension against purified water was measured. The interfacial tension was measured with the force tensiometer SIGMA 702 (KSV Instruments LTD).

In general, when the interfacial tension is 5 mN/m or less, capsules are likely to crack in the production process, and encapsulation is often difficult.

For this reason, the criteria for encapsulation capability (i.e., capability to form capsules without trouble) was set at 5 mN/m, a sample having an interfacial tension of 5 mN/m or more was rated A, and a sample having an interfacial tension of less than 5 mN/m was rated B in terms of entrapment efficiency.

The results are shown below.

### Experiment C1: 1-menthol + diethyl malonate

**[Table 59]**

| | | | | |
|---|---|---|---|---|
| I-Menthol (pbw) | 80 | 70 | 60 | 50 |
| Diethyl malonate (pbw) | 20 | 30 | 40 | 50 |
| Entrapment efficiency | A | A | A | A |

When diethyl malonate was used as a solvent to dissolve l-menthol, samples containing 80 to 50 wt% l-menthol had an interfacial tension of 5 mN/m or more and are seemingly capable of being encapsulated.

### Experiment C2: 1-menthol + diethyl succinate

**[Table 60]**

| | | | | |
|---|---|---|---|---|
| l-Menthol (pbw) | 80 | 70 | 60 | 50 |
| Diethyl succinate (pbw) | 20 | 30 | 40 | 50 |
| Entrapment efficiency | A | A | A | A |

When diethyl succinate was used as a solvent to dissolve l-menthol, samples containing 80 to 50 wt% l-menthol had an interfacial tension of 5 mN/m or more and are seemingly capable of being encapsulated.

### Experiment C3: 1-menthol + diethyl sebacate

**[Table 61]**

| | | | |
|---|---|---|---|
| I-Menthol (pbw) | 70 | 60 | 50 |
| Diethyl sebacate (pbw) | 30 | 40 | 50 |
| Entrapment efficiency | A | A | A |

When diethyl sebacate was used as a solvent to dissolve l-menthol, samples containing 70 to 50 wt% l-menthol had an interfacial tension of 5 mN/m or more and are seemingly capable of being encapsulated.

### Experiment C4: 1-menthol + diethyl succinate + MCT

**[Table 62]**

| | | |
|---|---|---|
| I-Menthol (pbw) | 70 | 60 |
| Diethyl succinate (pbw) | 15 | 10 |
| MCT (pbw) | 15 | 30 |
| Entrapment efficiency | A | A |

When diethyl succinate and the MCT were used as a solvent to dissolve l-menthol, samples containing 70 to 60 wt% l-menthol had an interfacial tension of 5 mN/m or more and are seemingly capable of being encapsulated.

### Experiment C5: 1-menthol + diethyl succinate + dodecanol + MCT

**[Table 63]**

| | | | | | | |
|---|---|---|---|---|---|---|
| I-Menthol (pbw) | 80 | 80 | 75 | 75 | 70 | 65 |
| Diethyl succinate (pbw) | 10 | 15 | 10 | 15 | 15 | 15 |
| Dodecanol (pbw) | 10 | 5 | 15 | 10 | 10 | 10 |
| MCT (pbw) | 0 | 0 | 0 | 0 | 5 | 10 |
| Entrapment efficiency | A | A | A | A | A | A |

When diethyl succinate, dodecanol, and the MCT were used as a solvent to dissolve l-menthol, samples containing 80 to 65 wt% l-menthol had an interfacial tension of 5 mN/m or more and are seemingly capable of being encapsulated.

### Experiment C6: 1-menthol + diethyl succinate/diisobutyl adipate/diethyl sebacate + 1-dodecanol

**[Table 64]**

| | | | | | | | | | |
|---|---|---|---|---|---|---|---|---|---|
| I-Menthol (pbw) | 70 | 65 | 60 | 70 | 65 | 60 | 70 | 65 | 60 |
| Diethyl succinate (pbw) | 26 | 31 | 36 | 0 | 0 | 0 | 0 | 0 | 0 |
| Diisobutyl adipate (pbw) | 0 | 0 | 0 | 26 | 31 | 36 | 0 | 0 | 0 |
| Diethyl sebacate (pbw) | 0 | 0 | 0 | 0 | 0 | 0 | 26 | 31 | 36 |
| 1-Dodecanol (pbw) | 4 | 4 | 4 | 4 | 4 | 4 | 4 | 4 | 4 |
| Entrapment efficiency | A | A | A | A | A | A | A | A | A |

When 1-dodecanol plus diethyl succinate, diisobutyl adipate, or diethyl sebacate were used as a solvent to dissolve l-menthol, samples containing 70 to 60 wt% l-menthol had an interfacial tension of 5 mN/m or more and are seemingly capable of being encapsulated.

### Experiment C7: 1-menthol + MCT

**[Table 65]**

| | | | | | | | |
|---|---|---|---|---|---|---|---|
| I-Menthol (pbw) | 70 | 65 | 60 | 55 | 50 | 45 | 35 |
| MCT (pbw) | 30 | 35 | 40 | 45 | 50 | 55 | 65 |
| Entrapment efficiency | A | A | A | A | A | A | A |

When the MCT was used as a solvent to dissolve l-menthol, samples containing 70 to 35 wt% l-menthol had an interfacial tension of 5 mN/m or more and are seemingly capable of being encapsulated.

### Experiment C8: 1-menthol + limonene + MCT

**[Table 66]**

| | | | | | |
|---|---|---|---|---|---|
| I-Menthol (pbw) | 80 | 70 | 70 | 65 | 60 |
| d-Limonene (pbw) | 20 | 5 | 10 | 5 | 5 |
| MCT (pbw) | 0 | 25 | 20 | 30 | 35 |
| Entrapment efficiency | A | A | A | A | A |

When d-limonene and MCT were used as a solvent to dissolve l-menthol, samples containing 80 to 60 wt% l-menthol had an interfacial tension of 5 mN/m or more and are seemingly capable of being encapsulated.

### Experiment C9: 1-menthol + benzyl alcohol + MCT

**[Table 67]**

| | | | | | |
|---|---|---|---|---|---|
| I-Menthol (pbw) | 80 | 70 | 70 | 65 | 60 |
| Benzyl alcohol (pbw) | 20 | 5 | 10 | 5 | 5 |
| MCT (pbw) | 0 | 25 | 20 | 30 | 35 |
| Entrapment efficiency | A | A | A | A | A |

When benzyl alcohol and the MCT were used as a solvent to dissolve l-menthol, samples containing 80 to 60 wt% l-menthol had an interfacial tension of 5 mN/m or more and are seemingly capable of being encapsulated.

### Experiment C10: 1-menthol + peppermint oil + MCT

**[Table 68]**

| | | | | | |
|---|---|---|---|---|---|
| I-Menthol (pbw) | 80 | 70 | 70 | 65 | 60 |
| Peppermint oil (pbw) | 20 | 5 | 10 | 5 | 5 |
| MCT (pbw) | 0 | 25 | 20 | 30 | 35 |
| Entrapment efficiency | A | A | A | A | A |

When peppermint oil and the MCT were used as a solvent to dissolve l-menthol, samples containing 80 to 60 wt% l-menthol had an interfacial tension of 5 mN/m or more and are seemingly capable of being encapsulated.

### Experiment C11: 1-menthol + spearmint oil + MCT

**[Table 69]**

| | | | | | |
|---|---|---|---|---|---|
| I-Menthol (pbw) | 80 | 70 | 70 | 65 | 60 |
| Spearmint oil (pbw) | 20 | 5 | 10 | 5 | 5 |
| MCT (pbw) | 0 | 25 | 20 | 30 | 35 |
| Entrapment efficiency | A | A | A | A | A |

When spearmint oil and the MCT were used as a solvent to dissolve l-menthol, samples containing 80 to 60 wt% l-menthol had an interfacial tension of 5 mN/m or more and are seemingly capable of being encapsulated.

### Experiment C12: 1-menthol + emulsifier + MCT

**[Table 70]**

| | |
|---|---|
| I-Menthol (pbw) | 60 |
| Organic acid monoglyceride (pbw) | 10 |
| MCT (pbw) | 30 |
| Entrapment efficiency | B |

When the organic acid monoglyceride and the MCT were used as a solvent to dissolve l-menthol, a sample containing 60 wt% l-menthol had an unmeasurably low interfacial tension (1 mN or less) and is seemingly incapable of being encapsulated.

### Encapsulation

The results of the encapsulation test above evidently indicate that the compositions of Experiments C1 to C11 are capable of being encapsulated, but further experiments confirmed that all the compositions can form capsules without trouble.

That is, all the compositions were successfully entrapped in seamless capsules (easily disintegratable capsules) by the drop method.

The capsules were designed to have a diameter of 3.4 mm (shell thickness: 50 µm, content mass: 19.3 mg).

The formula of the capsule shell was the same as that used in "Capsule Production Example 1" of Japanese Patent No. 6603817.

The capsules had a crush strength of 1,530 g and a crush deformation of 1.4 mm.

In addition to the type of capsule having a capsule diameter of 3.4 mm, the following four types of easily disintegratable capsules were also formed by a drop method. It should be noted that the formula of the capsule shell (outer layer) was the same among the capsules.
Capsule diameter: 2.8 mm, shell thickness: 57 µm, crush strength: 1,180 g, crush deformation: 1.5 mm
Capsule diameter: 3.0 mm, shell thickness: 48 µm, crush strength: 1,270 g, crush deformation: 1.6 mm
Capsule diameter: 3.5 mm, shell thickness: 48 µm, crush strength: 1,670 g, crush deformation: 1.8 mm
Capsule diameter: 4.0 mm, shell thickness: 45 µm, crush strength: 2,060 g, crush deformation: 2.0 mm

The physical properties of the capsules were measured or evaluated according to the following methods.

### Capsule crush strength and elasticity (crush deformation)

The crush strength of the capsules was measured using a rheometer CR-3000EX manufactured by Sun Scientific at room temperature (22 to 27°C) and 40 to 60% RH.

In the above measurement, the distance by which the capsule deformed until it crushed (the distance by which the capsule was compressed with the rheometer until the capsule crushed) was used as an index of the elasticity of the capsule.

### Capsule outer diameter

The outer diameter of the capsules was measured using a digital caliper manufactured by Mitutoyo Corporation (trade name: Quick Mini 25, model number: PK-0510SU, measurement range: 0 to 25 mm) at room temperature (22 to 27°C) and 40 to 60% RH.

### Capsule shell percentage

The percentage of the shell (shell percentage) was calculated as follows: shell percentage (%) = capsule shell mass / total capsule mass × 100.

The mass was measured using an electronic balance GX-200 manufactured by A&D Company, Limited.

### Capsule shell thickness

The thickness of the capsule shells (shell thickness) was measured using a digital microscope manufactured by Keyence Corporation (trade name: VHX-900, using a 10 µm calibration scale).

When each of the prepared capsules was picked and pressed with fingers, it broke easily with a snap, and the breaking sound and feel of the capsule were enjoyable. In addition, the flavor of l-menthol was enjoyable.

It will be understood that the shell of the capsule used for encapsulating the composition of the present invention may be a so-called plant-based capsule shell, which is free of gelatin, or a capsule shell containing gelatin.

The capsule prepared by encapsulating the composition of the present invention can be embedded in a tobacco filter, for example, but other applications are also conceivable. For example, such a capsule can be applied to an inhalation device for inhaling volatile ingredients without combustion, unlike conventional tobacco products involving combustion.

### INDUSTRIAL APPLICABILITY

The present invention provides an l-menthol-containing composition and others.

## Claims

1. A composition comprising l-menthol and at least one ingredient (X) selected from dicarboxylic acid esters, diol esters, monocarboxylic acid esters, esters of polyols having three or more hydroxy groups, esters of polycarboxylic acids having three or more carboxyl groups, polyol ethers, polyamines, and alcohols having six or more carbon atoms.

2. A composition comprising l-menthol and an ingredient (X) satisfying at least one selected from the following requirements (1) and (2):
(1) the ingredient (X) has a water/octanol partition coefficient (log P) of 10 or less; and
(2) the ingredient (X) has a hydrophilic-lipophilic balance (HLB) value of 12 or less.

3. A composition comprising l-menthol and at least one ingredient (X) selected from dicarboxylic acid esters, diol esters, monocarboxylic acid esters, esters of polyols having three or more hydroxy groups, esters of polycarboxylic acids having three or more carboxyl groups, polyol ethers, polyamines, and alcohols having six or more carbon atoms, wherein the ingredient (X) satisfies at least one selected from the following requirements (1) and (2):
(1) the ingredient (X) has a water/octanol partition coefficient (log P) of 10 or less; and
(2) the ingredient (X) has a hydrophilic-lipophilic balance (HLB) value of 12 or less.

4. The composition according to claim 2 or 3, wherein the ingredient (X) satisfies at least the requirement (1).

5. The composition according to any one of claims 2 to 4, wherein the ingredient (X) satisfies the requirements (1) and (2).

6. The composition according to any one of claims 2 to 5, wherein the ingredient (X) has a log P of 0.3 to 8 and/or an HLB value of 9 or less.

7. The composition according to any one of claims 1 to 6, wherein the ingredient (X) comprises at least one selected from dicarboxylic acid diesters, diol diesters, monocarboxylic acid esters, triol triesters, tricarboxylic acid triesters, diol diethers, and diamines.

8. The composition according to any one of claims 1 to 7, wherein the ingredient (X) comprises at least one selected from aliphatic dicarboxylic acid diesters, diesters of aliphatic diols having three or more carbon atoms, aliphatic monocarboxylic acid esters, aliphatic triol tri-C₁₋₆ fatty acid esters, aliphatic diol diethers, and N-alkyl substituted diamines.

9. The composition according to any one of claims 1 to 8, wherein the ingredient (X) comprises an aliphatic dicarboxylic acid diester.

10. The composition according to any one of claims 1 to 9, wherein the ingredient (X) comprises a diester of an aliphatic dicarboxylic acid having four or more carbon atoms.

11. The composition according to any one of claims 1 to 10, wherein the ingredient (X) comprises at least one selected from succinic acid diesters, adipic acid diesters, and sebacic acid diesters.

12. The composition according to any one of claims 1 to 11, wherein the ingredient (X) comprises at least one selected from 1-decanol, 1-dodecanol, 1-hexadecanol, and benzyl alcohol.

13. The composition according to any one of claims 1 to 12, wherein the ingredient (X) comprises at least one selected from 1-dodecanol and 1-hexadecanol.

14. The composition according to any one of claims 1 to 13, wherein the composition comprises
an alcohol having six or more carbon atoms, and
a fat or oil and/or an ingredient (X) other than the alcohol having six or more carbon atoms.

15. The composition according to any one of claims 1 to 14, wherein the composition comprises
at least one selected from C₁₀₋₁₆ alkanols and aralkyl alcohols, and
at least one selected from dicarboxylic acid esters and fats or oils.

16. The composition according to any one of claims 1 to 15, wherein the concentration of the l-menthol is 20 mass% or more.

17. The composition according to any one of claims 1 to 16, wherein the concentration of the l-menthol is 30 mass% or more.

18. The composition according to any one of claims 1 to 17, wherein the concentration of the l-menthol is 50 mass% or more.

19. The composition according to any one of claims 1 to 18, wherein the amount of the ingredient (X) is 80 mass% or less of the combined amount of the l-menthol and the ingredient (X) .

20. The composition according to any one of claims 1 to 19, wherein the amount of the ingredient (X) is 50 mass% or less of the combined amount of the l-menthol and the ingredient (X) .

21. The composition according to any one of claims 1 to 20, further comprising a fat or oil.

22. The composition according to any one of claims 1 to 21, wherein the composition is a pharmaceutical composition.

23. A capsule having a core and a shell, wherein the core is formed of the composition according to any one of claims 1 to 22.

24. A filter at least containing a capsule, wherein the capsule has a core and a shell, and wherein the core is formed of the composition according to any one of claims 1 to 22.

25. A tobacco product containing the composition according to any one of claims 1 to 22.

26. An inhalation device containing the composition according to any one of claims 1 to 22.

27. The inhalation device according to claim 26, wherein the inhalation device is a smoking device.

28. The tobacco product or inhalation device according to any one of claims 25 to 27, wherein the tobacco product or inhalation device contains the capsule according to claim 23 or the filter according to claim 24.

29. A perfumery or cosmetic product containing the composition according to any one of claims 1 to 22.

30. The perfumery or cosmetic product according to claim 29, wherein the perfumery or cosmetic product is an air freshener, an oral care product, or a cosmetic product.

31. A food or drink product containing the composition according to any one of claims 1 to 22.

32. The food or drink product according to claim 31, wherein the food or drink product is in the form of a capsule.

33. A pharmaceutical product containing the composition according to any one of claims 1 to 22.

34. The pharmaceutical product according to claim 33, wherein the pharmaceutical product is a gastric peristalsis inhibitor.

35. An agent for enhancing dissolution of l-menthol, comprising at least one ingredient (X) selected from dicarboxylic acid esters, diol esters, monocarboxylic acid esters, esters of polyols having three or more hydroxy groups, esters of polycarboxylic acids having three or more carboxyl groups, polyol ethers, polyamines, and alcohols having six or more carbon atoms.

36. An agent for enhancing dissolution of l-menthol, comprising an ingredient (X) satisfying at least one selected from the following requirements (1) and (2):
(1) the ingredient (X) has a water/octanol partition coefficient (log P) of 10 or less; and
(2) the ingredient (X) has a hydrophilic-lipophilic balance (HLB) value of 12 or less.

37. An agent for enhancing dissolution of l-menthol, comprising an ingredient (X) comprising at least one selected from dicarboxylic acid esters, diol esters, monocarboxylic acid esters, esters of polyols having three or more hydroxy groups, esters of polycarboxylic acids having three or more carboxyl groups, polyol ethers, polyamines, and alcohols having six or more carbon atoms, wherein the ingredient (X) satisfies at least one selected from the following requirements (1) and (2):
(1) the ingredient (X) has a water/octanol partition coefficient (log P) of 10 or less; and
(2) the ingredient (X) has a hydrophilic-lipophilic balance (HLB) value of 12 or less.

38. A method for enhancing dissolution of l-menthol, comprising bringing l-menthol into contact with an ingredient (X) comprising at least one selected from dicarboxylic acid esters, diol esters, monocarboxylic acid esters, esters of polyols having three or more hydroxy groups, esters of polycarboxylic acids having three or more carboxyl groups, polyol ethers, polyamines, and alcohols having six or more carbon atoms.

39. A method for enhancing dissolution of l-menthol, comprising bringing l-menthol into contact with an ingredient (X) satisfying at least one selected from the following requirements (1) and (2):
(1) the ingredient (X) has a water/octanol partition coefficient (log P) of 10 or less; and
(2) the ingredient (X) has a hydrophilic-lipophilic balance (HLB) value of 12 or less.

40. A method for enhancing dissolution of l-menthol, comprising bringing l-menthol into contact with an ingredient (X) comprising at least one selected from dicarboxylic acid esters, diol esters, monocarboxylic acid esters, esters of polyols having three or more hydroxy groups, esters of polycarboxylic acids having three or more carboxyl groups, polyol ethers, polyamines, and alcohols having six or more carbon atoms, wherein the ingredient (X) satisfies at least one selected from the following requirements (1) and (2):
(1) the ingredient (X) has a water/octanol partition coefficient (log P) of 10 or less; and
(2) the ingredient (X) has a hydrophilic-lipophilic balance (HLB) value of 12 or less.

41. A method for pulmonary l-menthol delivery, comprising using the capsule according to claim 23 or the filter according to claim 24.

42. A method for pulmonary l-menthol delivery, comprising using the tobacco product, the inhalation device, and/or the perfumery or cosmetic product according to any one of claims 25 to 28.
